# EUROPEAN PATENT APPLICATION

(11) **EP 1 808 134 A2**
(43) Date of publication of application: **18.07.2007**
(21) Application number: 07000619.2
(22) Date of filing: 12.01.2007
(51) Int. Cl.: A61B 17/04, A61B 17/32, A61B 18/14

(54) **Endoscopic overtube**

(30) Priority: 13.01.2006 US 331938; 21.02.2006 US 358257; 23.02.2006 US 360198; 08.03.2006 US 371565
(71) Applicant: OLYMPUS MEDICAL SYSTEMS CORP., Tokyo (JP)
(72) Inventor: Miyamoto, Manabu, c/o Olympus Med. Syst. Corp., Tokyo (JP); Kogasaka, Takahiro, c/o Olympus Med. Syst. Corp., Tokyo (JP); Yamatani, Ken, c/o Olympus Med. Syst. Corp., Tokyo (JP); Dejima, Takumi, c/o Olympus Med. Syst. Corp., Tokyo (JP); Takeuchi, Saori, c/o Olympus Med. Syst. Corp., Tokyo (JP); Matsuno, Kiyotaka, c/o Olympus Med. Syst. Corp., Tokyo (JP)
(74) Representative: von Hellfeld, Axel

(57) **Abstract**

An overtube according to this invention includes: an insertion part (5), that is inserted into a subject and has a lumen (3), through which a device insertion part of a device (endoscope 2) for performing a medical procedure inside a body of the subject is removably inserted, the insertion part being inserted into the subject; a tissue incising part (6) that is disposed at a distal end side of the insertion part so as to cross the lumen and incises a tissue of the subject; a manipulating member (electrode manipulating wires 7A, 7B) which is connected to the tissue incising part and is disposed in a manner enabling advancing and retracting with respect to the insertion part; a manipulating part (electrode manipulating part 8) for manipulating the manipulating member to advance and retract with respect to the lumen; and a needle manipulating part (10) for manipulating a puncture needle (32A, 32B) and a pusher (35).

## Description

### BACKGROUND OF THE INVENTION

### Field of the Invention

This invention relates to an overtube and a medical procedure using the overtube that is performed via a natural orifice.

### Description of Related Art.

Laparoscopic operations are known in which, in performing a medical procedure of observing, treating, etc. an organ of the human body, instead of incising the abdominal wall widely, a plurality of orifices are opened in the abdominal wall and procedures are performed upon inserting a laparoscope, forceps, and other treatment instruments into the orifices. Such procedure provides the benefit of lessening the burden placed on the patient because only small orifices need to be opened in the abdominal wall.

In recent years, methods of performing procedures upon inserting a flexible endoscope via the mouth, nose, anus, or other natural orifice of the patient have been proposed as methods of further reducing the burden on the patient. An example of such procedures is disclosed in United States Patent No. 5,458,131.

With this method, a flexible endoscope is inserted from the mouth of a patient, an opening is formed in the stomach wall, and a distal end part of the endoscope is fed into the abdominal cavity from the opening. Then while using the endoscope as a device for observing the interior of the abdominal cavity, desired procedures are performed inside the abdominal cavity using a treatment instrument inserted through the endoscope or a treatment instrument inserted from another opening.

However, in the above-described conventional method, when incising the wall of hollow organ in the abdominal cavity, it is necessary to form the opening in order to approach the endoscope into the abdominal cavity through the opening, by inserting incising instruments such as a high frequency treatment instrument into an instrument insertion passage of the endoscope, and by manipulating the incising instruments. Therefore, the manipulation becomes troublesome.

### SUMMARY OF THE INVENTION

An object of this invention is to provide an overtube that enable incision of tissue to be performed more readily in performing a medical procedure using an endoscope.

The present invention employs the following techniques in order to overcome the above-described drawbacks.

An overtube of the present invention is characterized by comprising: an insertion part, that is inserted into a subject and has a lumen, through which a device insertion part of a device for performing a medical procedure inside a body of the subject is removably insetted, the insertion part being inserted into the subject; and a tissue incising part that is disposed at a distal end side of the insertion part so as to cross the lumen and incises a tissue of the subject.

Furthermore, the overtube of the present invention is characterized in that the tissue incising part is disposed so as to allow withdrawing of the crossing state of said lumen.

Furthermore, the overtube of the present invention is characterized in that the tissue incising part can be removed from the insertion part.

Furthermore, the overtube of the present invention is characterized by further comprising: a manipulating member, connected to the tissue incising part and being disposed in a manner enabling advancing and retracting with respect to the insertion part; and a manipulating part for manipulating the manipulating member to advance and retract with respect to the lumen.

Furthermore, the overtube of the present invention is characterized in that the manipulating member is detachably connected to the manipulating part,

Furthermore, the overtube of the present invention is characterized in that the tissue incising part crosses a center of the lumen from an outer edge of the lumen in a direction orthogonal to an axial direction of the insertion part.

Furthermore, the overtube of the present invention is characterized in that hollow puncture needles, advancing and retracting along the lumen, are disposed at the distal end side of the insertion part.

Furthermore, the overtube of the present invention is characterized by further comprising: thread members extending from the tissue incising part to a proximal end of the insertion part, and a fastening member provided on distal ends of the thread members for fastening the thread members to a tissue of the subject in proximity to a targeted incision site.

Furthermore, the overtube of the present invention is characterized in that the fastening member comprises anchors attached to the distal ends of the thread members.

Furthermore, the overtube of the present invention is characterized in that the anchors are respectively held inside the puncture needles.

Furthermore, the overtube of the present invention is characterized in that two sets of the thread member in which the anchors attached to the distal end sides thereof, and two sets of said puncture needle are provided; and each of the two anchors retains on both sides of the targeted incision cite so as to situate the targeted incision cite therebetween.

Furthermore, the overtube of the present invention is characterized by further comprising a member for adjusting a gap between the anchors which are attached to the distal ends of the thread members by decreasing the gap to the predetermined value.

Furthermore, the overtube of the present invention is characterized in that the cutting instrument uses a high frequency,

Furthermore, the overtube of the present invention is characterized in that the cutting instrument has a tip, and a radius of curvature of the tip is set within a range of 0.05 mm to 0.5 mm.
Furthermore, the overtube of the present invention is characterized in that the puncture needle can be protrude out from an inside of the lumen and can be observed by an observation apparatus which is provided at the distal end side of the lumen.

Furthermore, the overtube of the present invention is characterized in that a magnetic body is disposed in the insertion part.

Furthermore, the overtube of the present invention is characterized by further comprising a holding portion that holds the tissue cutting portion at a position that is removed from a position traversing the first lumen.

Furthermore, the overtube of the present invention is characterized in that the holding portion includes puncture needles that are disposed so as to be freely advanced and retracted in the insertion portion and that are to puncture the tissue.

Furthermore, the overtube of the present invention is characterized in that the distal end portion of the insertion portion has a groove provided therein, and the tissue cutting portion is engaged so as to be retained in the groove by the puncture needle that traverses the groove.

Furthermore, the overtube of the present invention is characterized in that the puncture needles are structured to accommodate anchors that are to be retained in the tissue.

Furthermore, the overtube of the present invention is characterized by further comprising an expanded portion, wherein the expanded portion has a groove that is provided in the distal end portion of the insertion portion, and the holding portion is formed by extending an inner peripheral side of the distal end portion of the insertion portion so as to cover a portion of the groove of the expanded portion.

Furthermore, the overtube of the present invention is characterized in that the holding portion includes a tube that extends from one end side and the other end side of the tissue cutting portion and that passes through an operating member that extends toward the proximal end portion of the insertion portion by passing through the insertion portion.

Furthermore, the overtube of the present invention is characterized in that the manipulating member moves the tissue cutting portion from a first position removed from the position traversing the lumen to a second position traversing the lumen.

Furthermore, the overtube of the present invention is characterized in that the puncture needle is provided in an inside of said overtube that is defined by said lumen, and the overtube further comprising a needle manipulating part for retracting said puncture needle to the proximal end direction of said insertion portion.

Furthermore, the overtube of the present invention is characterized in that a tube that passes through the insertion portion is provided, and the tissue cutting portion which is held in the tube can be advanced with respect to the tube.

Furthermore, the overtube of the present invention is characterized by further comprising: a first lumen that is formed in the insertion portion and that allows freely inserting and removing a device for carrying out medical procedures in the subject; puncture needles that pass through the insertion portion, have a control member connected to the proximal end portion of the insertion portion, and puncture tissue; and second lumens that are provided in the insertion portion and through which puncture needles are passed; wherein the second lumens having on the distal end portion of the insertion portion openings through which distal ends of the puncture needles extend and retract, and the openings being provided on an inside of the insertion portion defined by the first lumen.

Furthermore, the overtube of the present invention is characterized in that an opening is formed on the distal end portion of the insertion portion so as to communicate with the first lumen in order to project the device in the forward insertion direction for insertion into the subject.

Furthermore, the overtube of the present invention is characterized by further comprising a guide portion that controls the projection direction of the puncture needles that project from the openings of the second lumens and that makes the puncture needles project in a center direction of the first lumen that is in cross-section substantially perpendicular to the direction in which the distal end portion of the insertion portion connects the proximal end portion of the Insertion portion.

Furthermore, the overtube of the present invention is characterized in that, at the distal end portion of the insertion portion, the second lumens are formed inside the distal end portion, and the centers of the openings of the second lumens are formed in the inner surface of the distal end portion of the first lumen more towards the proximal side than the opening of the insertion portion.

Furthermore, the overtube of the present invention is characterized in that a distal end area of the second lumens has a passage that angles the puncture needles from the proximal end portion to the distal end portion from the inner surface of the distal end portion that forms the first lumen to the direction of the center axis of the first lumen.

Furthermore, the overtube of the present invention is characterized in that an abutting portion formed at a distal end of the distal end portion of the insertion portion abuts the tissue when the tissue of the subject is sucked.

Furthermore, the overtube of the present invention is characterized in that each puncture needle has a passage therein that can accommodate an anchor that is to be disposed on an exit side of a punctured tissue.

Furthermore, the overtube of the present invention is characterized in that the puncture needles include a first puncture needle and a second puncture needle, each of the puncture needles being disposed at opposing positions across the approximate center of the first lumen.

Furthermore, the overtube of the present invention is characterized in that the anchors accommodated in the first puncture needle and the second puncture needle are connected together by a thread, and the distal end has a hole which accommodates a stopper portion provided on the thread.

Furthermore, the overtube of the present invention is characterized by further comprising: a first lumen that is formed in the insertion portion that has a distal end portion and a proximal end portion, and allows freely inserting and removing a device for carrying out medical procedures in the subject; puncture needles that pass through the insertion portion, have a control member connected to the proximal end portion of the insertion portion, and puncture tissue; and second lumens that are provided in the insertion portion and through which puncture needles are passed, the second lumens having on the distal end portion of the insertion portion openings through which distal ends of the puncture needles extend and retract, and the openings being provided on an inside of the insertion portion which forms the first lumen.

Furthermore, the overtube of the present invention is characterized by further comprising: an insertion portion having a distal end portion and a proximal end portion and at least the distal end portion thereof is inserted into an examination subject; a pair of lumen tubes formed in the insertion portion, each of the pair of lumen tubes having a distal end opening and a proximal end opening formed therein, and disposed such that the distal end opening is separated at the distal end portion of the insertion portion; an activation part disposed between the respective distal end openings of the pair of lumen tubes so as to be usable; a device that extends from both ends of the activation part and has an operation part that passes through the pair of lumen tubes so as to freely advance and retract; and a fastening member that releasably fastens proximal end portions of the pair of lumen tubes that are pulled from the proximal end portion of the insertion portion, and while the operation part is exposed by releasing one of the pair of lumen tubes, the released lumen tube can be moved relative to the operation part.

According to the present invention, it is possible to carry out the incision of tissue more readily in performing a medical procedure using an endoscope.

### BRIEF DESCRIPTION OF THE DRAWINGS

FIG. 1 is a schematic view of an entirety of an overtube according to a first embodiment.
FIG. 2 is a view of principal portions of the overtube according to the first embodiment.
FIG. 3 is a perspective view of a distal end part of the overtube according to the first embodiment.
FIG 4A is a sectional view taken along line III-III of FIG 3,
FIG 4B is a sectional view taken along line IV-IV of FIG 4A.
FIG 5 is a partially enlarged section of an electrode manipulating part of the overtube according to the first embodiment.
FIG 6A is an entire view of double T-bars used in the embodiment.
FIG 6B is a sectional view of a state in which the double T-bars are fitted into a puncture needle of the overtube according to the first embodiment.
FIG. 7 is a partial sectional view of a needle manipulating part of the overtube according to the first embodiment.
FIG. 8 is a sectional view of a portion near an endoscope lock button of the overtube according to the first embodiment.
FIG. 9 is an entire schematic view of an endoscope as an example of a device used for the overtube according to the first embodiment.
FIG 10 is a flowchart of a medical procedure according to the first embodiment.
FIG 11 is a view for describing a state of inserting the endoscope into the overtube according to the first embodiment.
FIG 12 is a view for describing a state of introducing the overtube to an incision target site according to the first embodiment.
FIG. 13 is a view for describing a state of suctioning a portion of a stomach wall into the overtube according to the first embodiment.
FIG. 14 is a view for describing a state of puncturing the suctioned stomach wall by means of the puncture needle of the overtube according to the first embodiment.
FIG 15 is a view for describing a state of insufflation of an abdominal cavity by feeding of air from a hypodermic needle according to the first embodiment.
FIG 16 is a view for describing a state of releasing anchors of the double T-bars from the puncture needle according to the first embodiment.
FIG 17 is a view for describing a state of incising the suctioned stomach wall by means of the incising electrode of the overtube according to the first embodiment.
FIG. 18 is a view of FIG. 17 viewed from a direction rotated by 90 degrees.
FIG. 19 is a view for describing a state of inserting the endoscope into the abdominal cavity according to the first embodiment.
FIG 20 is a view for describing a state of pulling and constricting a string of the double T-bars that have been placed according to the first embodiment.
FIG. 21 is a schematic drawing showing the overall overtube according to a second embodiment.
FIG. 22 is a perspective view showing the distal end portion of the overtube according to a second embodiment
FIG. 23 is a drawing for explaining the state in which the endoscope has been inserted in the overtube according to a second embodiment.
FIG 24 is a drawing for explaining the state in which the stomach wall that has suction applied has been punctured by the puncture needles of the overtube while the abdomen is insufflated with air that has been delivered from the injection needle according to a second embodiment.
FIG. 25 is a drawing for explaining the state in which the anchors have been released from the puncture needles according to a second embodiment.
FIG. 26 is a drawing for explaining the state in which the stomach wall has been cut by the cutting electrode of the overtube while pulling the proximal end sides of the thread members according to a first embodiment.
FIG 27 is a drawing showing FIG 26 after being rotated 90 degrees.
FIG. 28 is a drawing for explaining a procedure for suturing the opening portion that has been cut by using two retained anchors and the thread members extending therefrom in a medical procedure according to a second embodiment.
FIG 29 is a drawing for explaining a procedure for suturing the opening portion that has been cut by using two retained anchors and the thread members extending therefrom according to a second embodiment.
FIG 30 is a cross-sectional view showing a modified example of the distal end portion of an overtube according to a second embodiment.
FIG 31 is a drawing for explaining the state before the stomach wall has been cut by the cutting instrument while pulling the proximal end sides of the thread members of the overtube according to a third embodiment.
FIG 32 is a drawing for explaining the state after the stomach wall has been cut by the cutting instrument while pulling the proximal end sides of the thread members of the overtube according to a third embodiment.
FIG 33 is a perspective view of the cutting instrument according to a third embodiment.
FIG 34 is a perspective view of another cutting instrument according to a third embodiment.
FIG. 35 is a perspective view of the tip of the cutting instrument according to a third embodiment.
FIG. 36 is a side view of the cutting instrument shown in FIG. 35.
FIG. 37 is a frontal view of the cutting instrument shown in FIG. 35.
FIG. 38 is a side view of another tip of a cutting instrument according to a third embodiment.
FIG. 39 is a frontal view of the tip of a cutting instrument that is shown in FIG. 38.
FIG. 40 is a perspective view of a cutting instrument according to a fourth embodiment.
FIG 41 is a drawing for explaining the state before the stomach wall is cut by the cutting instrument while pulling the proximal end sides of the thread members of the overtube according to a fourth embodiment.
FIG. 42 is a drawing for explaining the state after the stomach wall has been cut by the cutting instrument while pulling the proximal end sides of the thread members of the overtube according to a fourth embodiment.
FIG 43 is a perspective view of another cutting instrument according to a fourth embodiment.
FIG 44 is a drawing for explaining the state before the stomach wall is cut by another cutting instrument while pulling the proximal end sides of the thread members of the overtube according to a fourth embodiment.
FIG 45 is a drawing for explaining the state after the stomach wall has been cut by another cutting instrument while pulling the proximal end sides of the thread members of the overtube according to a fourth embodiment.
FIG 46 is a perspective view of an endoscope according to a fifth embodiment.
FIG 47 is a drawing for explaining the state before the stomach wall is cut by a cutting instrument while pulling the proximal end sides of the thread members of the overtube according to a fifth embodiment.
FIG. 48 is a drawing for explaining the state after the stomach wall has been cut by another cutting instrument while pulling the proximal end sides of the thread members of the overtube according to a fifth embodiment.
FIG 49 is a view of principal portions of an overtube according to a sixth embodiment.
FIG 50 is a sectional view taken along line A-A of FIG 49.
FIG 51 is a flow chart according to the sixth embodiment.
FIG 52 is a view for describing a state of making an endoscope inserting part protrude from the overtube according to the sixth embodiment.
FIG. 53 is a view for describing a state of using the endoscope inserting part as a guide and using a second magnet of the overtube to move the overtube inside an abdominal cavity from the state shown in FIG. 52.
FIG. 54 is a view for describing a state of making the endoscope inserting part protrude along with the overtube from an orifice according to the sixth embodiment.
FIG. 55 is a view for describing a state of using the second magnet of the overtube to move the endoscope inserting part and the overtube inside the abdominal cavity from the state shown in FIG 54.
FIG. 56 is a view for describing a state of using the first magnet of the overtube to support the overtube inside the abdominal cavity according to the sixth embodiment.
FIG. 57 is a view for describing a state of using the first magnet and third magnet of the overtube to change the direction of the overtube inside the abdominal cavity according to the sixth embodiment.
FIG 58 is a perspective view showing the distal end portion of the overtube according to a seventh embodiment.
FIG 59 is a cross-sectional view along B.B in FIG. 58.
FIG. 60 is a cross-sectional view along D-D in FIG 58.
FIG 61 is a drawing for explaining the state in which the overtube according to a seventh embodiment is introduced to the targeted incision cite.
FIG 62 is a drawing for explaining the state in which a portion of the stomach wall is sucked into the overtube according to a seventh embodiment.
FIG 63 is a drawing for explaining the state in which the abdomen has been insufflated by supplying air from an injection needle of the overtube according to a seventh embodiment.
FIG. 64 is a drawing for explaining the state in which the sucked stomach wall has been punctured by a puncture needle of the overtube according to a seventh embodiment.
FIG. 65 is a drawing for explaining the state in which the anchors of the double T bar have been ejected from the puncture needles of the overtube according to a seventh embodiment.
FIG. 66 is a drawing for explaining the state in which the sucked stomach wall has been cut by the cutting electrode of the overtube according to a seventh embodiment.
FIG 67 is a view of FIG. 66 after being rotated 90º.
FIG 68 is a front view of the distal end portion showing a modified example of the overtube according to a seventh embodiment.
FIG. 69 is a cross-sectional view of the distal end portion showing a modified example of the overtube according to a seventh embodiment.
FIG 70 is a drawing for explaining another method in which the cutting electrode is exposed during cutting, and is a cross-sectional view through a plane that crosses the axial line of the distal end portion.
FIG 71 is a schematic diagram showing the entire overtube for another method in which the cutting electrode is exposed during cutting.
FIG 72 is a drawing in which the electrode tube has been detached and the electric controlling wires have been exposed.
FIG 73 is a drawing in which the electrode controlling wires and the electrode tube have been moved relative to each other.
FIG 74 is a drawing in which one electrode controlling wire has been pulled by the action of FIG 73.
FIG 75 is a drawing in which the cutting electrode is drawn into the electrode tube.
FIG 76 is a perspective view of a modification example of principal portions of an overtube.
FIG 77 is a perspective view of another modification example of principal portions of an overtube.
FIG. 78 is a perspective view in which a scale and a scale detecting member have been provided on the proximal handle.
FIG 79 is a drawing for explaining the operation of the scale detecting member.
FIG 80 is a drawing showing a structure in which an impedance measuring device is provided and the timing at which the cutting of the tissue has completed can be detected.
FIG 81 is a drawing of another embodiment of the holding portion, and is a cross-sectional view showing the holding portion that extends from the distal end side.
FIG. 82 is a drawing showing another embodiment of the holding portion, and is a cross-sectional view showing the holding portion that extends from the proximal end side.
FIG 83 is a cross-sectional view showing an example of a modification in which the needle lumens are established along the inside perimeter of the lumen.

### DETAILED DESCRIPTION OF THE INVENTION

### First Embodiment

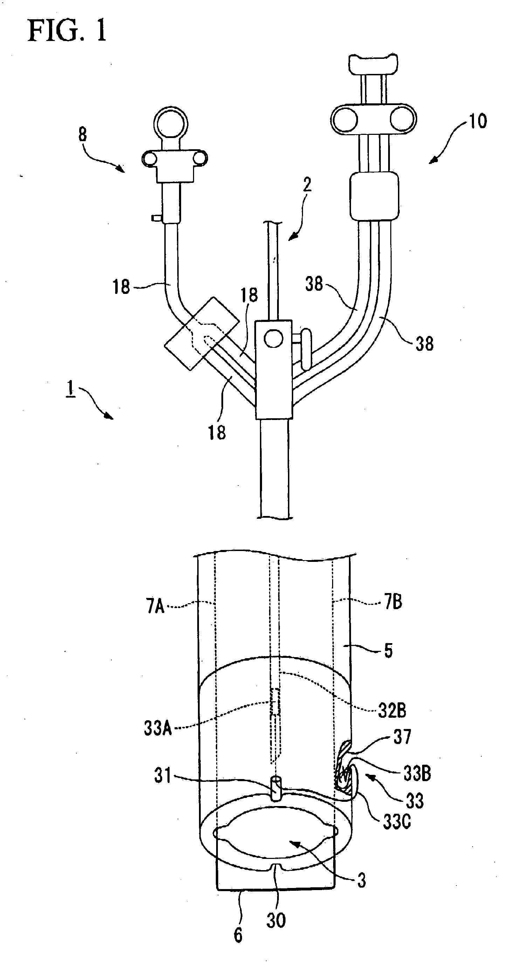

A first embodiment according to this invention shall now be described with reference to FIGS. 1 to 20.

An overtube 1 according to this embodiment is used as a guide tube for inserting, into a body, an endoscope or other device, for carrying out a medical procedure inside a body and being equipped with an insertion device part that is inserted inside a subject (to simplify the description, the device may be referred to simply as "device" or "endoscope" below). As shown in FIG 1, the overtube 1 includes: an insertion part 5 that is opened at a distal end, inserted into a stomach or other hollow organ or abdominal cavity, etc., of a patient (subject) and has a lumen 3, through which an endoscope 2, as one example of a device extending along an axial direction, is removably inserted; an incising electrode (tissue incising part) 6 that crosses a distal end side of the lumen 3, is disposed in the insertion part 5 so as to allow withdrawing of the crossing state, and incises an internal tissue of the patient; electrode manipulating wires (manipulating members) 7A and 7B that are each connected to the incising electrode 6 and are disposed in a manner enabling advancing and retracting with respect to the insertion part 5; an electrode manipulating part (manipulating part) 8 that is for manipulating the electrode manipulating wires 7A and 7B to advance and retract with respect to the lumen 3; and a needle manipulating part 10 that is for manipulating a puncture needle 32A, 32B, and a pusher 35 to be described later. Though in the present embodiment, the incising electrode 6 and the electrode manipulating wires 7A and 7B are arranged from a single wire, the incising electrode 6 and electrode manipulating wires 7A and 7B may be arranged as separate members that are connected to each other. Also, the incising electrode 6 may be disposed so as not to be able to advance and retract with respect to the insertion part 5.

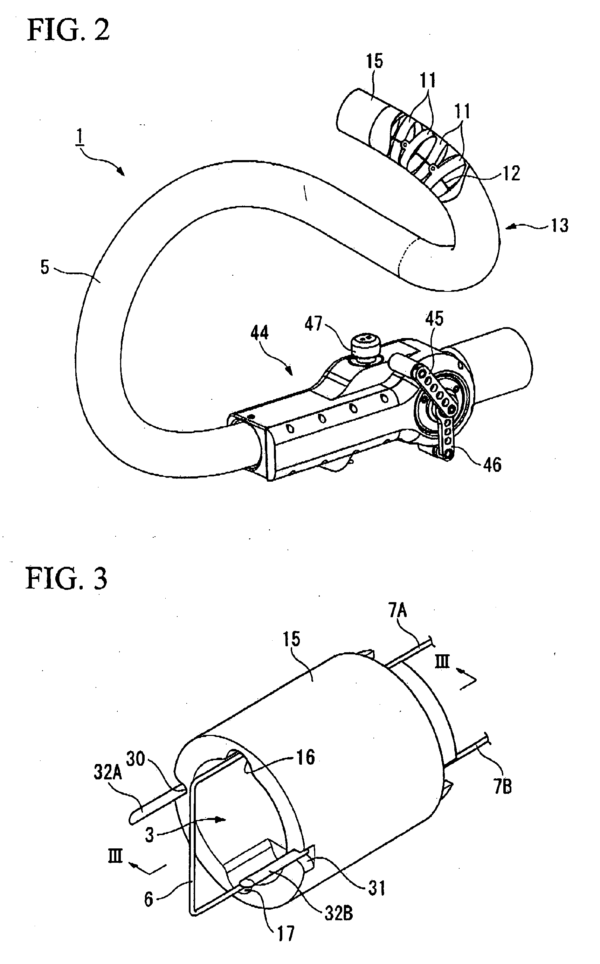

As shown in FIG. 2, the insertion part 5 is elongated and flexible, and as with a normal flexible endoscope, a bending part 13, in which a plurality of joint rings 11 are connected along bending wires 12, is disposed at a distal end side of the insertion part 5, Here, instead of providing the bending part 13 that is actively bended by manipulation by an operator, the insertion part may be arranged in a tube-like shape with flexibility and to be bended passively in accordance with a bending state of the endoscope, etc. A distal end part 15 having a short pipe shape is disposed further at the distal end of the bending part 13 as shown in FIG. 3.

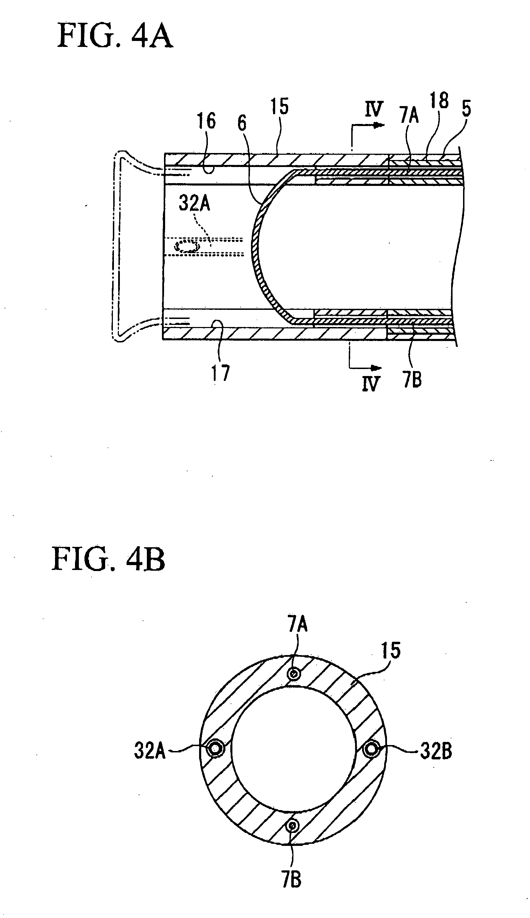

The incising electrode 6 is, for example, a stainless steel wire to which high-frequency electricity can be energized and is disposed so as to cross a center of the lumen 3 in a direction orthogonal to an axial direction of the insertion part 5. That is, as shown in FIGS. 3 and 4, one end side of the incising electrode 6 is inserted through a first inner groove 16, formed on an outer edge of the lumen 3 that is an inner surface of the distal end part 15 (in other words, an inner periphery of the distal end part 15 that defines the lumen 3), and is connected to the electrode manipulating wire 7A. The other end side of the incising electrode 6 is inserted through a second inner groove 17, formed on the outer edge of lumen 3 (in other words, the inner periphery of the distal end part 15 that defines the lumen 3) at a position substantially symmetrically across the center of the lumen 3 from the first inner groove 16, and is connected to the electrode manipulating wire 7B. The incising electrode 6 is formed so that its length is longer than the inner diameter of the lumen 3, and as shown in FIG. 4, the incising electrode 6 is accommodated in the lumen 3 with a bended state, and is movable along the first inner groove 16 and second inner groove 17. Though in the embodiment shown in FIG. 4, the length of the incising electrode 6 is set longer than the inner diameter of the lumen 3, this invention is not restricted thereto, and the length of the incising electrode 6 may be set (for example, to a length substantially equal to the inner diameter of the lumen 3) so that the incising electrode 6 is accommodated in the lumen 3 with a non-bended state.

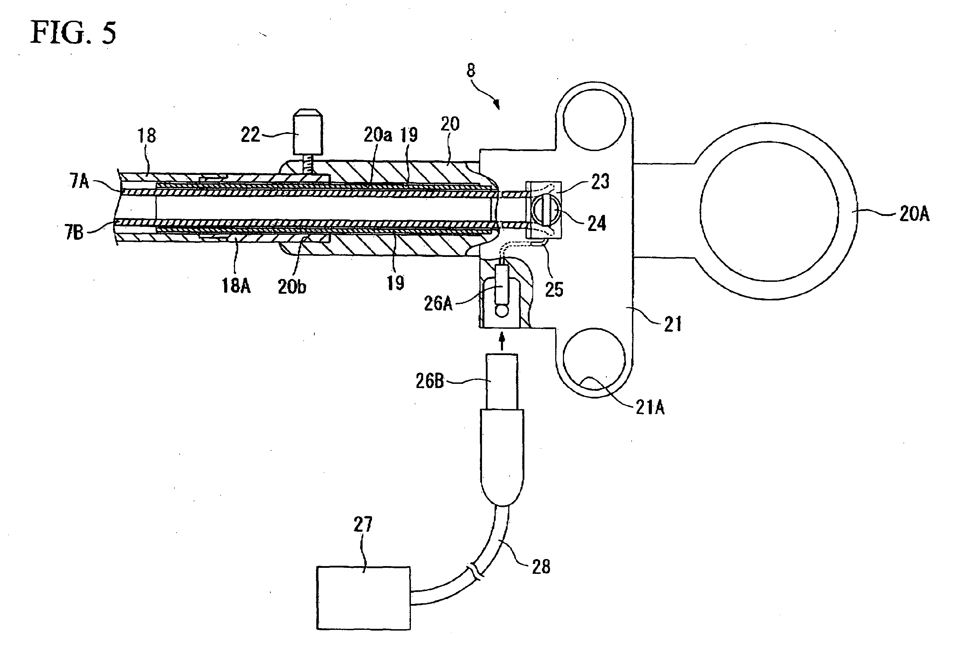

The electrode manipulating wires 7A and 7B are inserted through an electrode tube 18, The electrode tube 18 is provided with a single tube at a proximal end side that protrudes outside the insertion part 5. As shown in FIG 1, the electrode tube 18 branches into two at a middle portion, and are connected at a distal end to the distal end part 15, so that the electrode manipulating wires 7A and 7B are accommodated separately in the insertion part 5. As shown in FIG. 5, the proximal ends of the electrode manipulating wires 7A and 7B are inserted through a single, rigid manipulating pipe 19 disposed to protrude from a distal end of a manipulating handle 21 to be described later.

As shown in FIGS. 1 and 5, the electrode manipulating part 8 includes a manipulating body 20, which is connected to the proximal end of the electrode tube 18, and a manipulating handle 21, which is disposed to be able to advance and retract freely with respect to the manipulating body 20. The manipulating body 20 is provided with an insertion hole 20a, through which the electrode manipulating wires 7A and 7B and the manipulating pipe 19 are inserted. At a distal end of the manipulating body 20 is formed an engagement hole 20b, which engages with a rigid part 18A disposed at the proximal end of the electrode tube 18, and the electrode tube 18 is fixed to the engagement hole 20b by a screw 22. A finger ring 20A is disposed at a proximal end of the manipulating body 20.

A connection plate 23 is disposed at the manipulating handle 21. The connection plate 23 is electrically connected to end parts of the electrode manipulating wires 7A and 7B inserted through the manipulating pipe 19. A fixing screw 24 is disposed at the connection plate 23, and by screwing the fixing screw 24 into the connection plate 23, the electrode manipulating wires 7A and 7B are fixed and electrically connected. The connection plate 23 is electrically connected via an electric wiring 25 to a connection terminal 26A disposed in the manipulating handle 21. A connection terminal 26B, disposed at a distal end of a power supply cord 28 that extends from a high-frequency power supply 27, is detachably attached to the connection terminal 26A. The manipulating handle 21 is also provided with finger rings 21A.

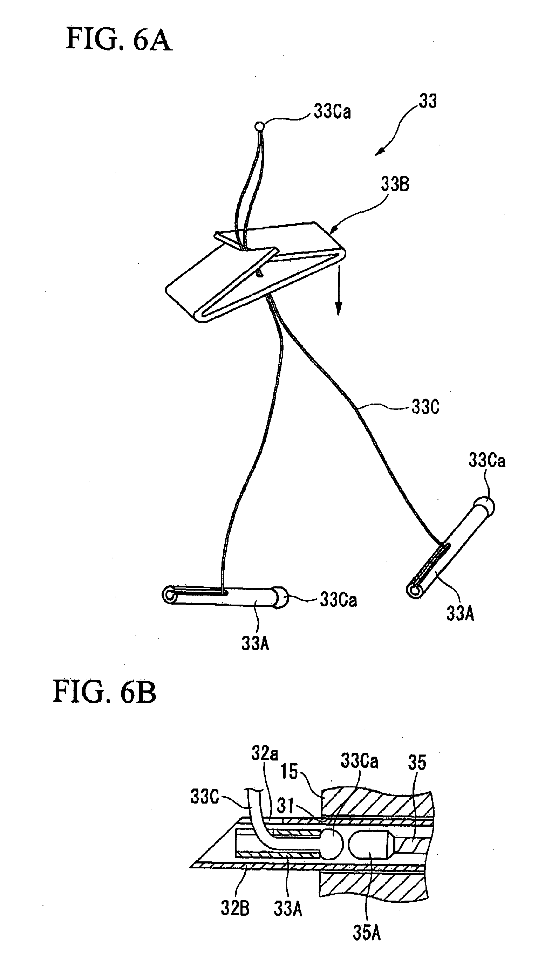

On an outer surface of the distal end part 15 of the overtube 1, a first outer groove 30 and a second outer groove 31 are formed from a middle portion to the distal end of the distal end part 15 at positions orthogonal to a direction joining the first inner groove 16 and the second inner groove 17. The two puncture needles (hollow needles) 32A and 32B, which advance and retract along the lumen 3, are movably disposed in advancing and retracting directions in the first outer groove 30 and the second outer groove 31, respectively. Two anchors 33A of double T-bars 33, shown in FIG 6A, are respectively held inside the respective puncture needles 32A and 32B as shown in FIG. 6B,

The double T-bars 33 have two sutures 33C, one end side of each of which is passed through a substantially triangular stopper 33B. At one end, the sutures 33C are bound together to form a large diameter part 33Ca. Each of the other ends of the sutures 33C is fixed to the anchors 33A. Each anchor 33A has a cylindrical shape with a slit formed at an end, and the suture 33C is inserted in the longitudinal direction of the interior of anchor 33A through the slit. The large diameter part 33Ca that has greater diameter than that of the anchor 33A is formed at the other end of the suture 33C. The stopper 33B has a hole, through which the sutures 33C are passed, at a center in the longitudinal direction of an elongated, thin plate member. The respective ends in the longitudinal direction of the stopper 33B are folded obliquely and sandwich the sutures 33C. The respective ends in the longitudinal direction of the stopper 33B are cut to notches of triangular shape. With the stopper 33B, the respective ends are folded back obliquely so that the notches intersect and thereby sandwich the sutures 33C. The sutures 33C thus do not fall off from between the ends. When the large diameter part 33Ca of the sutures 33C is pulled in a direction away from the stopper 33B, the respective end parts of the stopper 33B spread apart slightly. The stopper 33B thus allows movement of the sutures 33C in this direction. Meanwhile, when a large diameter part 33Ca at the anchor 33A side of a suture 33C is pulled, a tendency for the suture 33C to move in the direction indicated by the arrow in FIG 6A arises. However, since the respective ends of the stopper 33B close and grasp the sutures 33C in this process, the suture 33C does not move.

As shown in FIG 6B, the pusher 35 is movably disposed in advancing and retracting directions in the interior of the respective puncture needles 32A and 32B. As shown in FIG 1, the stopper 33B of the double T-bars 33 is accommodated inside a hole (referred to hereinafter simply as "hole") 37 formed from a proximal end side to the distal end side of a side face of the insertion part 5.

The puncture needles 32A and 32B and pushers 35 are respectively accommodated in two outer sheaths 38. Each of the two outer sheaths 38 is inserted through the insertion part 5 and has a distal end connected to the distal end part 15. A slit 32a, through which a suture 33C of the double T-bars 33 is inserted, is formed at a distal end of each of the puncture needles 32A and 32B. A rigid, pushing member 35A is disposed at a distal end of the pusher 35.

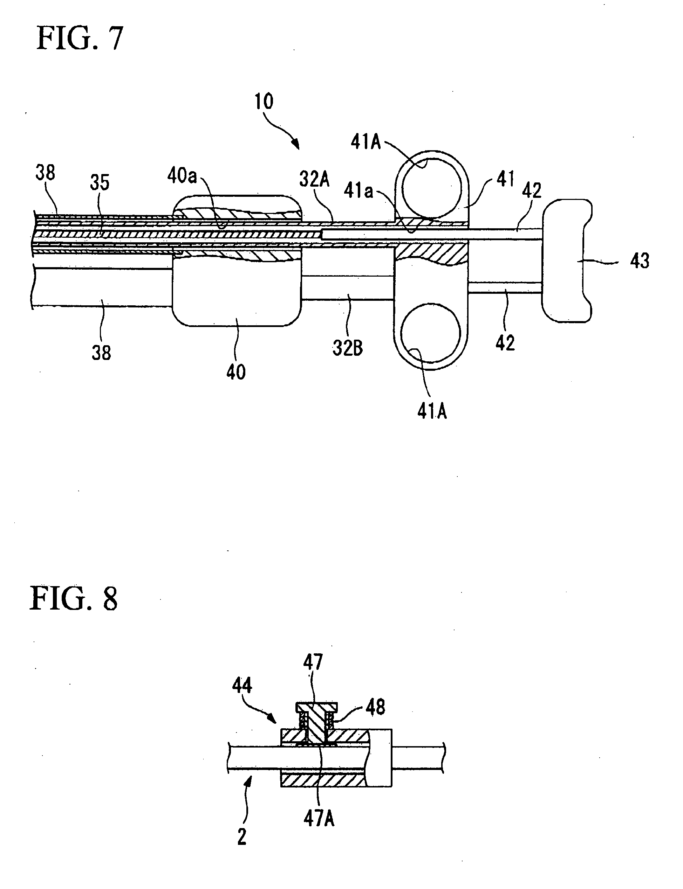

As shown in FIGS. 1 and 7, the needle manipulating part 10 includes: a sheath holding part 40, connected to the proximal ends of the two outer sheaths 38; a needle manipulating handle 41, connected to proximal ends of the two puncture needles 32A and 32B that have been passed in a manner enabling advancing and retracting through through-holes 40a formed in the sheath holding part 40; and a pusher connection part 43 that connects end portions of rod-like, rigid parts 42, which are passed in a manner enabling advancing and retracting through through-holes 41a formed in the needle manipulating handle 41 and are connected to proximal ends of the two pushers 35, to each other. The needle manipulating handle 41 is provided with finger rings 41A. Each of the needle manipulating handle 41 and the pusher connection part 43 may be divided into two parts so as to enable the two puncture needles 32A and 32B and the two pushers 35 to be manipulated independently of each other.

As shown in FIG 2, a proximal handle 44 having a larger diameter than the insertion part 5, is disposed at the proximal end of the insertion part 5 of the overtube 1. The proximal handle 44 includes a bending lever 45, a bending lock lever 46, and an endoscope lock button 47. The bending lever 45 is connected to the proximal ends of the bending wires 12 for performing bending manipulation of the bending part 13. The bending lock lever 46 is used for fix the position of the bending lever 45 at an arbitrary position. The endoscope lock button 47 is used for fix the endoscope 2 with respect to the lumen 3 upon insertion of the endoscope 2 through the lumen 3.

The distal ends of the bending wires 12 are fixed to the distal end part 15, and in the present embodiment, the two bending wires 12 are inserted through the interior of the insertion part 5 and the distal ends thereof are fixed to portions of the distal end part 15 that substantially oppose each other across the center of the lumen 3. Though in this embodiment, two bending wires 12 are provided to enable bending of the bending part 13 in two directions, this invention is not limited thereto, and four bending wires 12 and two bending levers 45 may be provided as in a bending part of a known endoscope to enable bending of the bending part in four directions.

As shown in FIG 8, the endoscope lock button 47 has a pressing part 47A of wide width disposed at a distal end and which is normally urged in an outward radial direction of the proximal handle 44 by a spring 48. When the endoscope 2 must be fixed to the insertion part 5 upon being inserted through the interior, the endoscope lock button 47 is pressed inward in the radial direction so that the pressing part 47A presses and fixes the endoscope 2 in a relative manner by a frictional force. The endoscope lock button 47 may be arranged so as to oppositely release the frictional force when pressed.

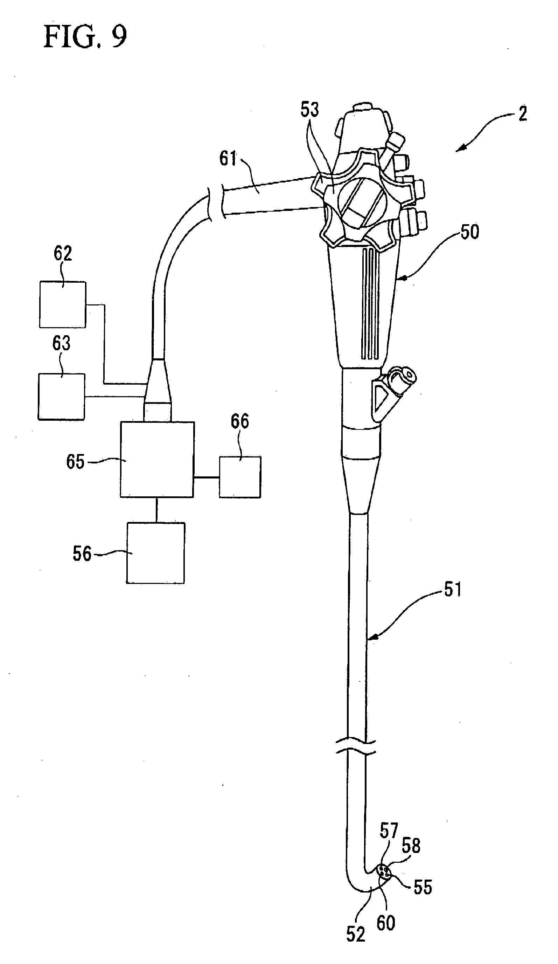

The endoscope 2, which is inserted into the overtube 1, is, for example, a flexible endoscope and, as shown in FIG 9, an endoscope inserting part 51, which is elongated and inserted into a patient's body, extends outward from an endoscope manipulating part 50 manipulated by an operator. An endoscope distal end part 52 of the endoscope inserting part 51 can be bended by manipulating an angle knob 53 disposed at the endoscope manipulating part 50. At the endoscope distal end part 52 are disposed an objective lens 55, a distal end face of an optical fiber 57 that guides light from a light source device 56 disposed outside the body, and distal end openings of channels 58 and 60. The channel 58 is a duct that is connected via a universal cable 61 to an air/water feeding device 62 or a suction device 63 disposed outside the body and is used to supply or drain fluid to or from inside the body. The channel 60 is a duct for inserting and removing a treatment instrument and is disposed at a position of six o'clock to eight o'clock of the endoscope inserting part 51. The number of treatment instrument channels is not restricted to one and, for example, two treatment instrument channels may be provided. An observation image inputted into the objective lens 55 is displayed on a monitor 66 via a control unit 65.

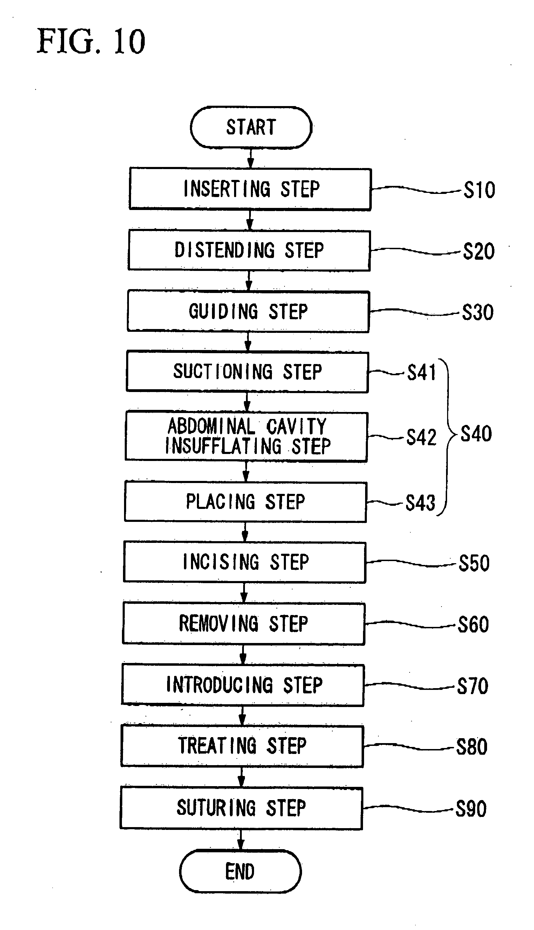

Actions of the present embodiment shall now be described in line with a medical procedure performed via a natural orifice using the overtube 1 as shown by the flow chart of FIG 10. In the following description, it shall be deemed that an incision target site T is located on an anterior wall of a stomach ST, and a surgical procedure of inserting the endoscope 2 into the stomach (hollow organ) ST from a mouth M of a patient PT and performing treatment upon forming an opening SO in the stomach wall and inserting the insertion part 5 of the endoscope 2 into an abdominal cavity AC shall be described. Also, though in the embodiment described below, the endoscope 2 is introduced as a device into the body from the mouth M of the patient PT and made to approach the abdominal cavity AC upon forming the opening SO in the anterior wall of the stomach ST, the natural orifice from which the endoscope 2 is introduced is not restricted to the mouth M and may be another natural orifice, such as the anus, nose, etc. Furthermore, though the forming of the opening SO in the anterior wall of the stomach ST is desirable, this invention is not restricted thereto, and an opening may be formed on a wall of the esophagus, small intestine, large intestine or other hollow organ (hollow organ) besides the stomach ST into which a device is introduced via a natural orifice.

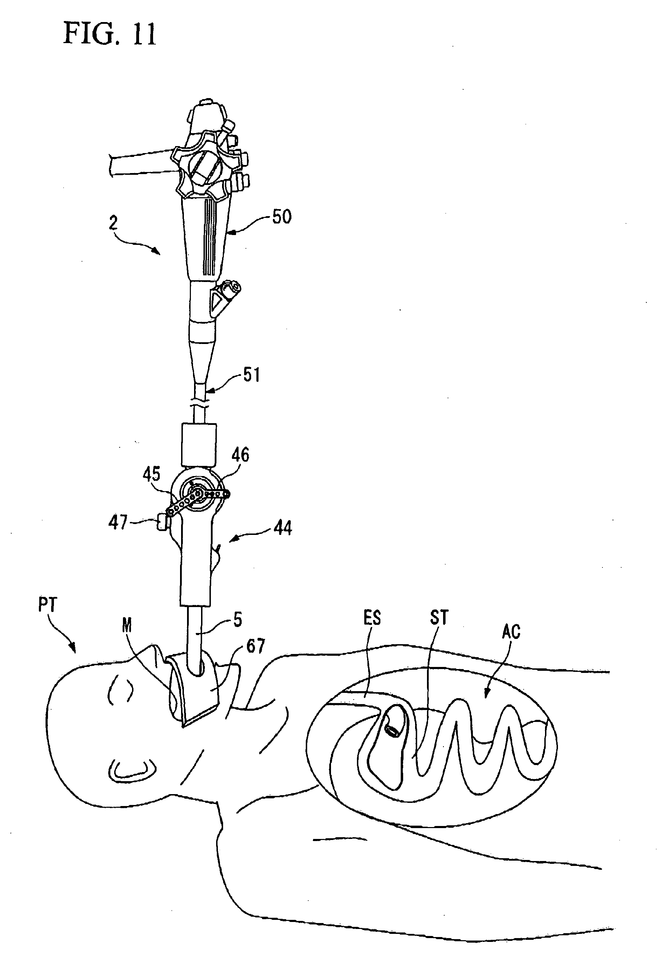

First, with the patient PT being made to lie in a supine position, an inserting step (S10) of inserting the endoscope 2 through the lumen 3 in the insertion part 5 of the overtube 1 and inserting the insertion part 5 of the overtube 1 and the endoscope 2 into the stomach (hollow organ) ST from the mouth M of the patient PT while observing the interior of the body cavity by means of an endoscopic image is performed. As shown in FIG. 11, a mouthpiece 67 is fitted onto the mouth of the patient PT and the overtube 1 and the endoscope 2 are inserted, with the endoscope 2 being inserted through the interior of the lumen 3, into the esophagus ES from the mouthpiece 67. It shall be deemed that the incising electrode 6 and the puncture needles 32A and 32B are all accommodated and positioned at initial positions inside the distal end part 15.

Next, in a distending step (S20), air is supplied from the air/water feeding device 62 via the channel 58 of the insertion part 5 to inflate the stomach ST.

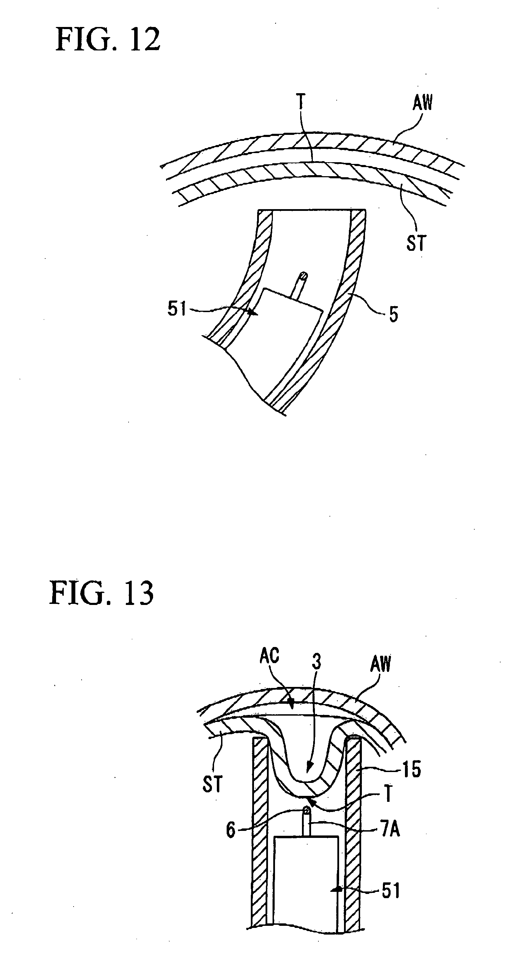

A guiding step (S30) of guiding the insertion part 5 of the overtube 1 to the incision target site T while checking the incision target site T using the endoscope 2, which is also an observation device, is then performed. First, after inserting the endoscope inserting part 51 of the endoscope 2 into the stomach ST, the angle knob 53 is manipulated to bring the distal end of the endoscope inserting part 51 dose to the incision target site T while observing the interior of the stomach ST via the objective lens 55, disposed at the endoscope inserting part 51. Then with the incision target site T being specified, the endoscope inserting part 51 is used as a guide to push the insertion part 5 of the overtube 1 and bring the distal end part 15 of the overtube 1 close to the incision target site T as shown in FIG. 12.

A needle moving step (S40), of advancing and retracting the puncture needles 32A and 32B, disposed at the distal end side of the insertion part 5, along the lumen 3, is then performed. First, in a suctioning step (S41), a stomach wall that includes the incision target site T is sucked in by the suction device 63 via the channel 58, with the distal end part 15 being put in contact with the stomach wall. In this process, a portion of the stomach wall is sucked into the distal end part 15 as shown in FIG. 13. A space is thereby secured between an outer side of the stomach wall and the abdominal cavity AC. The means for suctioning the stomach wall is not restricted to the method of using the channel 58 of the endoscope 2. For example, a space, formed between an inner surface of the lumen 3 of the overtube 1 and an outer periphery of the insertion part 5 of the endoscope 2 or other device inserted into the lumen 3, may be used as a suction channel and suction may be performed upon connecting the channel to the suction device 63. In this case, a valve (not shown) that controls the flow of fluid between the interior and exterior of the body may be provided in the formed space to further improve the suction effect.

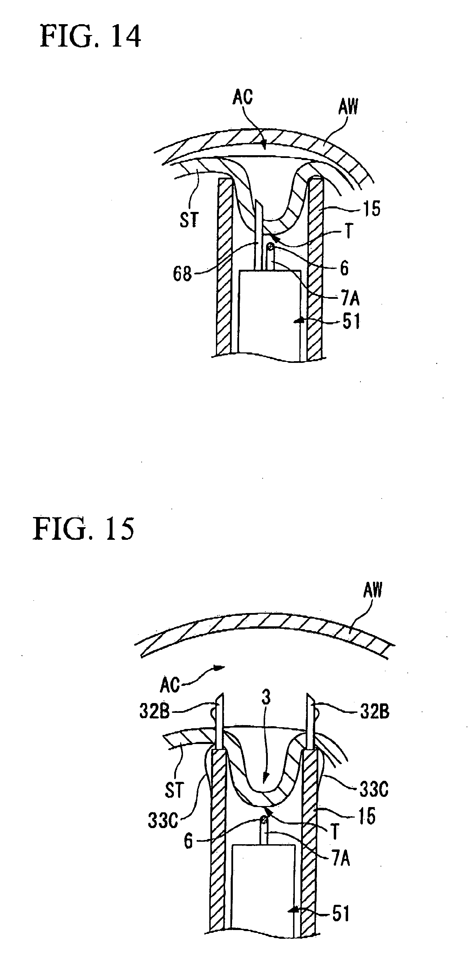

An abdominal cavity insufflating step (S42) is then performed. First, an injection needle 68 connected to the air/water feeding device 62 is inserted through the channel 60 of the endoscope 2. A distal end of the injection needle 68 is then protruded inside the distal end part 15, and as shown in FIG 14, pierced through the suctioned stomach wall and inserted to the abdominal cavity AC. Because the injection needle 68 is pierced with the stomach wall being sucked in and a space being secured with respect to the abdominal cavity AC, just the stomach wall can be punctured reliably. Air is then fed into the abdominal cavity AC via the injection needle 68 to insufflate the abdominal cavity AC so that the stomach ST and the abdominal cavity AC separate.

The injection needle 68 preferably has a needle length of approximately 12 mm and more preferably has a bendable distal end to enable piercing of the center of the suctioned stomach wall. In this case, a bended injection needle has a bending tendency at a distal end and has a bending wire (not shown) that passes from the distal end toward a proximal side in an inward radial direction of the bending tendency. Here, since the channel 60 of the endoscope 2 is disposed at a position of six o'clock to eight o'clock of the endoscope inserting part 51, the incision site is approached from an upward angle in incising the anterior stomach wall of the stomach ST that is preferable as the incision site. Since the bending wire thus faces the center due to the bending tendency following the bended state of the insertion part 5 of the overtube 1, the center of the stomach wall can be punctured reliably by pulling the bending wire toward the proximal side. In the process of feeding air, the interior of the abdominal cavity AC may be maintained at an appropriate pressure by monitoring and automatic control of the feed air pressure.

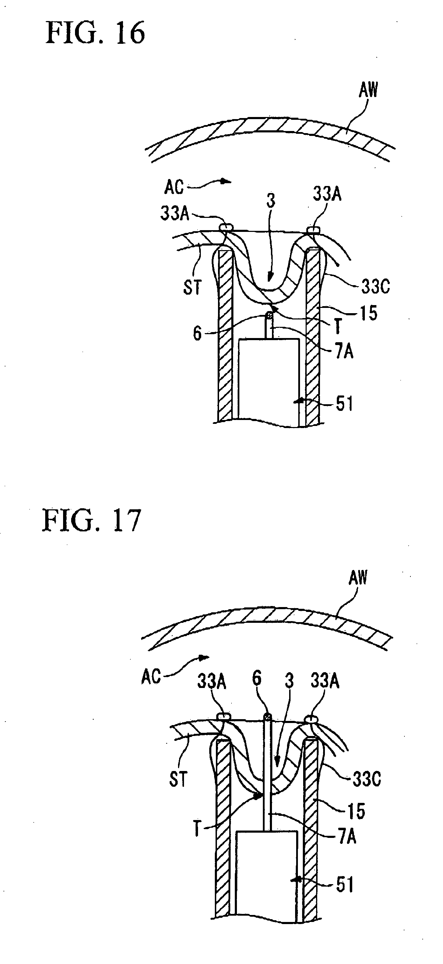

A placing step (S43) is then performed. First, the needle manipulating handle 41 is advanced in the direction of the sheath holding part 40 while holding the sheath holding part 40 to make the puncture needles 32A and 32B protrude from the first outer groove 30 and the second outer groove 31, respectively, of the distal end part 15 and pierce the stomach wall as shown in FIG. 15. From this state, the pusher connection part 43 is advanced with respect to the needle manipulating handle 41 to move the pushers 35 toward the distal ends of the puncture needles 32A and 32B. In this process, the anchors 33A of the double T-bars 33 are pressed by the pushers 35 and delivered out from inside the puncture needles 32A and 32B and into the interior of the abdominal cavity AC as shown in FIG 16. Here, since the hole 37 is formed so that it is directed from the proximal end side toward the distal end side of the insertion part 5, unintended falling off of the stopper 33B of the double T-bars 33 is prevented. Here, since the abdominal cavity AC is insufflated to secure a space with respect to the stomach wall, just the stomach can be punctured.

After the anchors 33A of the double T-bats 33 have been released, the pusher connection part 43 is retracted with respect to the needle manipulating handle 41, and furthermore, the needle manipulating handle 41 is retracted with respect to the sheath holding part 40 to respectively accommodate the puncture needles 32A and 32B inside the first outer groove 30 and the second outer groove 31 again. In this process, the two anchors 33A of the double T-bars 33 are put in a T-like state by the bending tendencies of the sutures 33C. Thereafter, by holding and drawing the sheath holding part 40 towards the proximal side, the puncture needles 32A and 32B are removed from the distal end part 15 and by furthermore drawing the puncture needles out from the overtube 1, the bending property of the bending part 13 is secured.

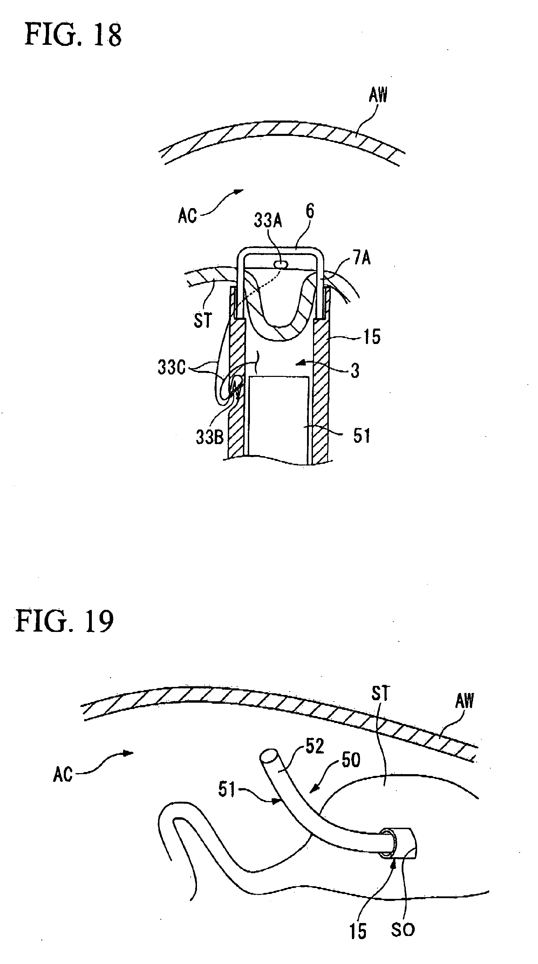

An incising step (S50) is then performed. First, it is checked whether the connection terminal 26A of the electrode manipulating part 8 is connected to the connection terminal 26B of the power supply cord 28. Then, while supplying the high-frequency power from high-frequency power supply 27, the manipulating handle 21 is advanced with respect to the manipulating body 20 to make the incising electrode 6 protrude from the distal end part 15 and contact the stomach wall. In this process, since the electricity is supplied to the incising electrode 6 via the electrode manipulating wires 7A and 7B, the stomach wall is incised by the incising electrode 6 and the opening SO is formed in the stomach wall as shown in FIGS. 17 and 18. By continuing the suctioning of the stomach wall in this step, the position of placement of the double T-bars 33 and the incision position are put in an optimal state.

A removing step (S60) is then performed. Here, in order to remove the incising electrode 6 from inside the insertion part 5, the fixing screw 24 of the manipulating body 20 of the electrode manipulating part 8 is loosened. In this process, the electrode manipulating wires 7A and 7B separate from the connection plate 23 and the electrode manipulating wires 7A and 7B become severed. Then, for example, an end part of the electrode manipulating wire 7A is held and drawn toward the proximal side to move the electrode manipulating wire 7A through the lumen 3 to the proximal end side and move the electrode manipulating wire 7B through the lumen 3 to the distal end side. Eventually, the electrode manipulating wire 7B also moves around the distal end opening of the lumen 3 and toward the proximal end side. The incising electrode 6 is thereby drawn out along with the electrode manipulating wires 7A and 7B.

An introducing step (S70) is then performed. That is, as shown in FIG. 19, the endoscope inserting part 51 of the endoscope 2, which is also an operative device, is introduced into the abdominal cavity AC through the opening SO. If, in this process, relative movement of the insertion part 5 and the endoscope inserting part 51 must be restricted, the endoscope lock button 47 is pressed and contacted against the endoscope inserting part 51 to fix the movement of the endoscope inserting part 51 by the frictional force. Since the endoscope lock button 47 is provided, the endoscope lock button 47 can be manipulated to restrain relative movement of the endoscope 2 with respect to the overtube 1, and the overtube 1 and the endoscope 2 can thus be inserted into the body simultaneously. Also, since the task of inserting the endoscope 2 can be performed while holding the proximal handle 44 of the overtube 1, an operation, in which the insertion part 5 of the overtube 1 is supported by one hand of the operator and the proximal handle 44 is held by the other hand, is enabled, and the operability is thus more improved.

When the overtube 1 is introduced into the abdominal cavity AC through the opening SO, the site of placement of the anchors 33A of the double T-bars is set at the proximal side of the position of the hole 37 formed in the insertion part 5. The stopper 33B, accommodated inside the hole 37, is thus pulled in the direction to become detached from the hole 37 in accordance with the orientation of the hole 37 and the stopper 33B falls out of the hole 37.

After positioning, a treating step (S80) of performing observation, incision, cell sampling, suturing, or any of other various treatments (medical procedures) is carried out. After performing the treatment, the overtube 1 and the endoscope 2 are removed from the opening SO of the stomach wall.

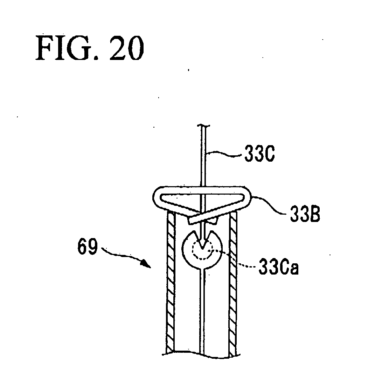

In a suturing step (S90), in removing the endoscope 2 from the opening SO, the large diameter part 33Ca of the sutures 33c is held and pulled with respect to the stopper 33B of the double T-bars 33, which had been placed in advance, by a ligating device 69, inserted through the channel 60 of the endoscope 2 as shown in FIG. 20. The opening SO is thereby sutured. Additional double T-bars 33, etc., are provided to perform further suturing if necessary. In this process, since the insufflation is performed in the process of placing the double T-bars 33 at the stomach wall, suturing by means of additional double T-bars 33 can be performed readily.

After suturing, the endoscope 2 is drawn out of the patient, the pressure applied to the abdominal cavity AC is released, and the surgical procedure is ended.

With the overtube 1, since the incising electrode 6 is disposed at the distal end side of the insertion part 5 and across the distal end side of the lumen 3, when the insertion part 5 is inserted into the stomach ST, the stomach wall can be incised without requiring a special treatment instrument for incision. Because, in this process, tissue is incised just by an amount corresponding to the length of the incising electrode 6 that crosses the lumen 3, the overtube 1 can be made to pass through with a light force and leakage at the outer periphery of the overtube 1 can be restrained preferably. Also, since the electrode manipulating wires 7A and 7B are removable with respect to the electrode manipulating part 8, the incising electrode 6 can be removed along with the electrode manipulating wires 7A and 7B from the insertion part 5.

Thus, in making the endoscope 2 protrude out from the lumen 3, the incising electrode 6 will not be an obstruction, and upon inserting the endoscope 2 through the lumen 3, the endoscope 2 can be advanced into the abdominal cavity AC beyond the incised tissue. Furthermore, in making the device (endoscope 2 in the embodiment) that has been inserted through the lumen 3 protrude and advance from the distal end of the overtube 1 after incising tissue and forming the opening, the task of withdrawing the incising electrode 6 from the path of the device, the task of drawing out the overtube 1 once from within the body to remove the incising electrode 6, etc., can be omitted. Consequently, the surgical procedure time from the forming of the opening in the stomach wall to the introducing of the endoscope 2 into the abdominal cavity AC can be shortened.

Also, since the incising electrode 6 is connected to the electrode manipulating wires 7A and 7B, which can be manipulated to advance and retract with respect to the lumen 3, the incising electrode 6 can be advanced and retracted with respect to the lumen 3 without performing a manipulation of advancing and retracting the entirety of the insertion part 5. That is, by advancing and retracting of the electrode manipulating part 8, the incising electrode 6 can be advanced and retracted with respect to the stomach wall to perform incision. In this process, because the incision is performed by passing high-frequency electricity through the incising electrode 6, the incision can be performed more safely with a small force.

Also, before incising and forming an opening in a wall of a hollow organ (the stomach wall in the embodiment), the double T-bars 33 can be placed, and in inserting the puncture needles 32A and 32B through the stomach wall, puncture can be performed preferably without the stomach wall moving away. Furthermore, the double T-bars 33 can be placed before opening formation (before suturing) to set up a state in which the double T-bars 33 are just constricted in the suturing process, and in suturing the opening after ending the medical procedure inside the abdominal cavity AG, the suturing of the opening can be performed more readily without insufflating the interior of the stomach. The suturing task can thus be performed more readily.

Also, because the direction in which the incising electrode 6 crosses the lumen 3 is orthogonal to the direction of joining the puncture needles 32A and 32B, the positions of puncturing by the puncture needles 32A and 32B can be separated from the incision location, and the double T-bars 33 can be placed at a position separated from the incision location by a distance that is appropriate for binding.

### Second embodiment

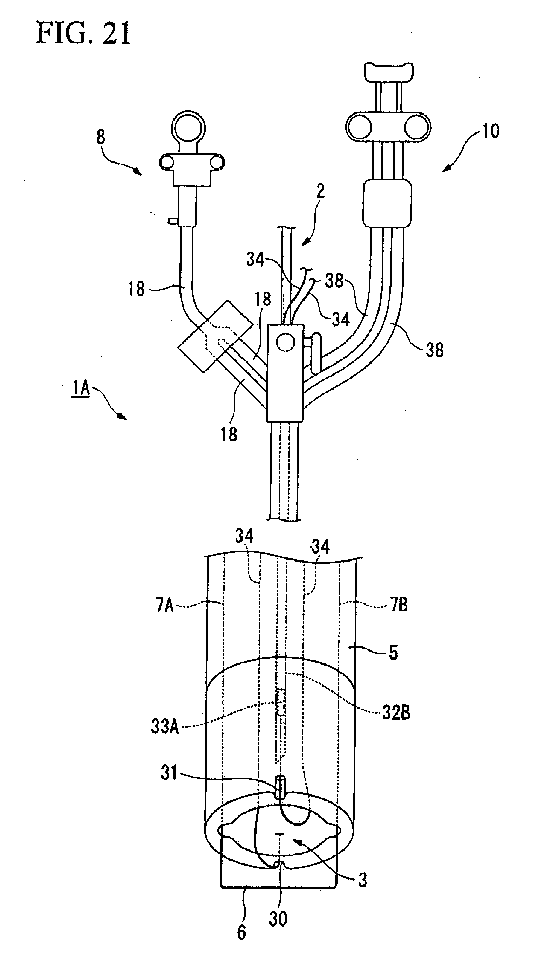

A second embodiment according to this invention shall now be described with reference to FIGS. 21 to 29. Note that below, identical structural elements are denoted by identical reference numerals, and redundant explanations thereof are omitted.

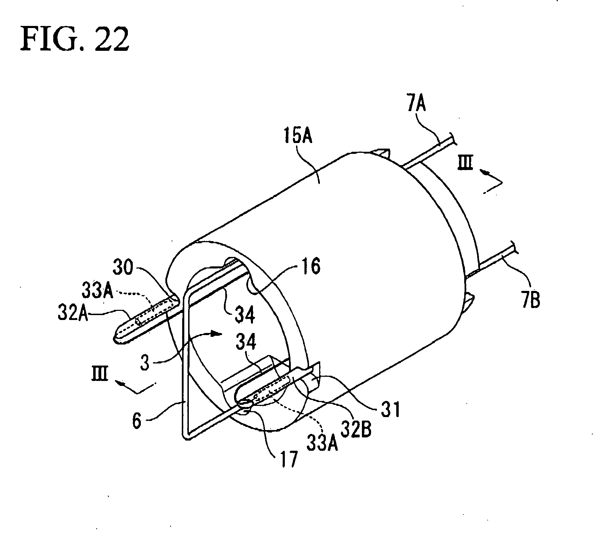

The second embodiment differs from the first embodiment described above on the point that instead of using the double T-bars 33, the proximal end sides of two pairs of thread members 34 bend back after reaching the edge of the distal end portion 15 of the overtube 1A, pass through the lumen 3 of the overtube 1A, and are exposed to the outside from the proximal end side opening of the overtube 1A as shown in figs 21 and 22.

Note that as necessary a disk having a diameter that is larger than the inner diameter of the overtube 1A may be attached or a rod member having a length that is longer than the inner diameter of the overtube 1A may be attached to the proximal end portion of the thread members 34 in order to prevent the proximal end sides of the thread members 33 from entering into the overtube 1A. The disk or the rod portions act as an engaging portion, and thereby the proximal end portions of the thread members 33 are prevented from being unintentionally drawn into the overtube 1A. In addition, it is possible to grasp the engaging portion when the proximal end portions of the thread members 34 are pulled.

Next, actions of the overtube 1A according to this embodiment shall now be described as in the first embodiment.

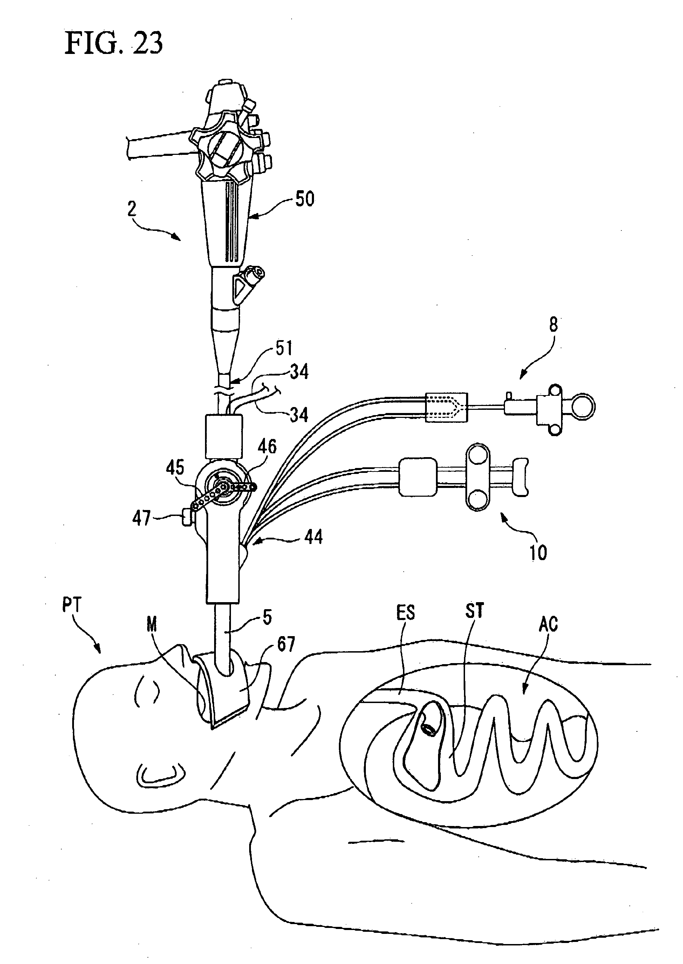

Firstly, as shown in FIG. 23, an inserting step (S10) is carried out, and then, a distending step (S20) and a guiding step (S30) are carried out, as in the first embodiment.

In a needle moving step (S40), a suctioning step (S41) and an insufflation step (S42) are carried out as in the first embodiment.

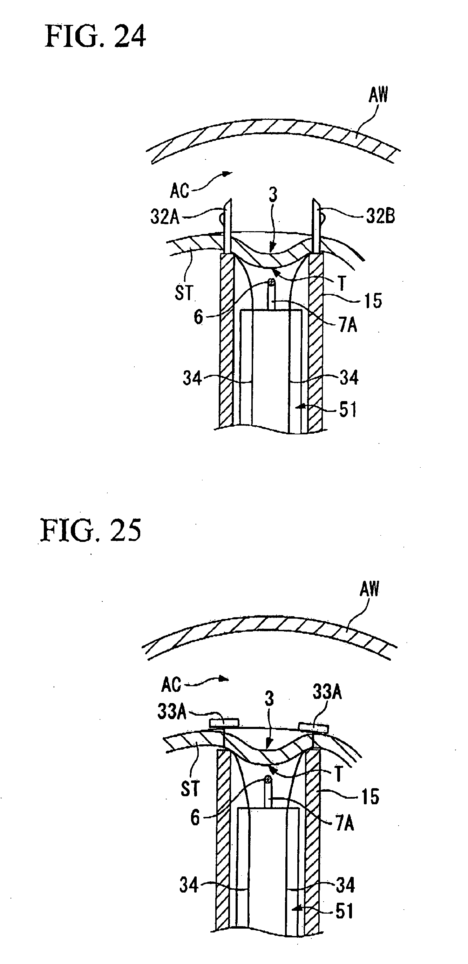

Then the process proceeds to a retention step (S43). Here, first the sheath grasping portion 40 is grasped and the needle controlling handle 41 is advanced toward the sheath grasping portion 40. Thereby, as shown in FIG 24, the puncture needles 32A and 32B puncture the stomach wall by respectively projecting from the first outer groove 30 and the second outer groove 31 of the distal end portion 15 toward the distal end side. Specifically, the puncture needles 32A and 32B, which hold the anchors 33A, penetrate both sides of the tissue such that the targeted incision site T is situated therebetween from the anterior side (the inside of the stomach wall) to reach the posterior side (the outside of the stomach wall). From this state, the pusher connecting portion 43 is advanced toward the needle controlling handle 41 to move the pushers 35 in the distal end direction of the puncture needles 32A and 32B. At this time, as shown in FIG. 25, the anchors 33A of the thread members 34 are pushed by the pusher 35 and delivered from the hollow portion of the puncture needles 32A and 32B into the abdominal cavity AC. Thereby, the two anchors 33A are each retained on the posterior side of the tissue in proximity to the targeted incision site T on both sides thereof such that the targeted incision site T is situated therebetween. Here, because the AC is insufflated and a space around the stomach wall is established, it is possible to puncture only the stomach wall.

After the anchors 33A of the thread members 34 have been delivered, the pusher connection portion 43 is retracted with respect to the needle controlling handle 41, and furthermore, the needle controlling handle 41 is retracted with respect to the sheath grasping portion 40 and thereby the puncture needles 32A and 32B are again accommodated in the first outer groove 30 and the second outer groove 31. At this time, the anchors 33A of the thread members 34 form a T-shape due to the bending of the thread members 33. Subsequently, the sheath grasping portion 40 is grasped, the puncture needles 32A and 32B are removed from the distal end portion 15 by pulling the distal side, and then removed from the overtube 1A. Thereby, the bending characteristics of the bending portion 13 are ensured.

Then the process proceeds to an incision step (S50). First, it is confirmed that the connecting terminal 26B of the power cord 28 is connected to the connecting terminal 26A of the electrode controlling portion 8. Then while supplying a high frequency power from a high frequency power source 27, the controlling handle 21 is advanced with respect to the controlling body portion 20, and the cutting electrode 6 is projected from the distal end portion 15 to abut the stomach wall. Simultaneously, the proximal end side of the two thread members 34, which are exposed from the proximal end side opening of the overtube, are pulled in a distal direction.

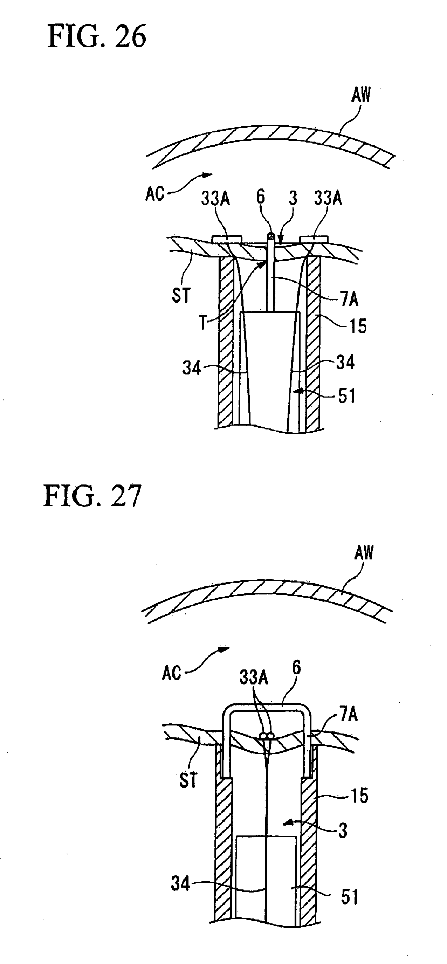

Thereby, the tissue that is present on both sides of the targeted incision site T can be pulled to the distal end side of the overtube 1, that is, to the cutting electrode 6 side, by each of the anchors 33A that are attached to the distal end of both thread members 34. Then the cutting electrode 6 is charged via the electrode controlling wires 7A and 7B, and thereby, as shown in FIGS. 26 and 27, the cutting electrode 6 cuts the stomach wall, and an opening SO is formed in the stomach wall.

Note that during this step also, by continuing to apply the suction to the stomach wall while simultaneously pulling the proximal end sides of the thread members 34 toward the proximal side, during the incision it is possible to prevent more completely the misalignment of the targeted incision site T.

Here, while the two thread members 34 are pulled in a proximal direction and the tissue that is present at both sides of the targeted incision site T is pulled toward the distal end side of the overtube 1A, the cutting electrode 6 may be pushed in the direction opposite to the direction in which the tissue is drawn (the forward direction of the overtube 1A) to cut the tissue. Specifically, when the cutting electrode 6 is disposed so as to move freely (advance and retract freely) with respect to the overtube 1A, it is possible to abut the cutting electrode 6 against the tissue and thereby cut the tissue while the tissue is being pulled.

Next, a removal step (S60) and an introduction step (S70) are carried out, and then, the process proceeds to a treatment step (S80) as in the first embodiment.

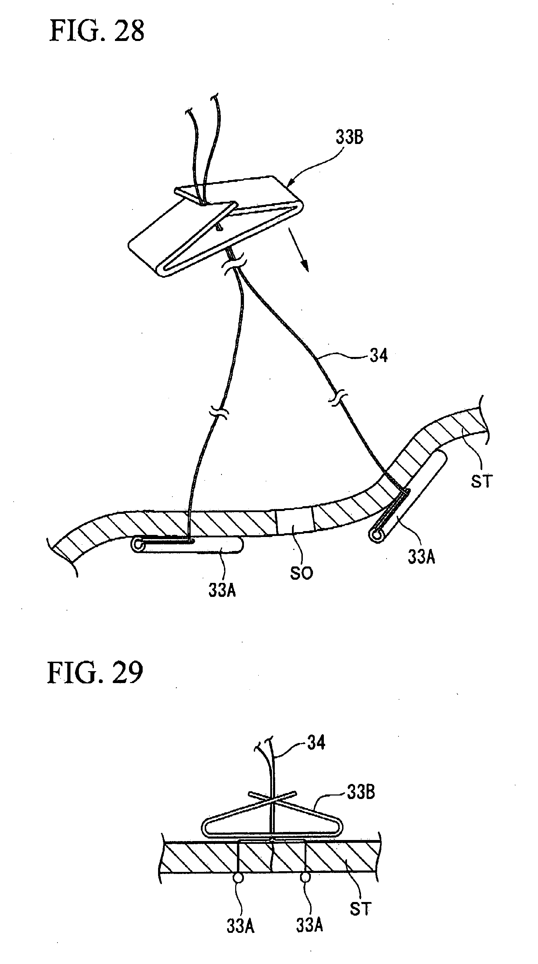

In the suturing step (S90), as shown in FIGS 28 and 29, the stopper 33B is engaged to the two thread members 34 that are exposed at the proximal end side of the overtube 1A.

Next, the stopper 33B will be explained. The stopper 33B has a hole through which the thread members 34 pass at the center of an elongated plate member in the longitudinal direction. Both end portions of the stopper 33B in the longitudinal direction can be bent back at an angle to hold the thread members 34. Both end portions of the stopper 33B in the longitudinal direction are cut into triangular shaped notches. Both end portions of the stopper 33B can be bent back at an angle such that the notches intersect, and the thread members 34 can be held there.

In addition, as shown in FIG. 28, the two thread members 33 pass through the hole at the center of the plate member, and at the same time, the stopper 33B engages the two thread members 34 such that the proximal end sides of the thread members 34 that have been passed through the hole are held by the intersecting portions of both ends of the plate member that have been bent back at an angle. In this state, the thread members 34 cannot fall out from between the proximal portions. In addition, when the proximal end sides of the thread members 34 are pulled in the direction of separation from the stopper 33B, both end portions of the stopper 33B open slightly. Thereby, the stopper 33B permits movement of the thread members 34 in this direction. In contrast, when the thread members 34 are pulled toward the distal end side with respect to the stopper 33B, the thread members 34 move in the direction indicated by the arrow in FIG 28. However, at this time, because both end portions of the stopper 33B close to clamp the thread members 33, the thread members 34 cannot move. That is, as shown in FIG 28, when the stopper 33B engages the thread members 34, the stopper 33B moves toward the distal end sides of the thread members 33, but does not move toward the proximal end side.

Then, the stopper 33B, which has been engaged in this manner, is moved up to the distal ends of the thread members 34. Here, a method of moving the stopper 33B up to the distal end positions of the thread members 34 may include, for example, covering the tube farther on the proximal end side than the portion engaged to the stopper 33B of the two thread members 34 and feeding the distal end of this tube toward the distal end sides of the thread members 34. Thereby, the stopper, which has been engaged to the distal end of the tube, moves integrally along with the tube.

As shown in FIG 29, by moving the stopper 33B up to the distal end sides of the thread members 34 in this manner, it is possible to bring the anchors 33B that are attached to the distal ends of the thread members 34 into proximity to each other, and thereby, it is possible to suture the opening SO.

Note that the method for suturing the tissue by using the two thread members 34 is not limited to a method in which such a stopper is used. A knot may be formed by tying together the sections of the two thread members 34 that extend from the proximal end side of the overtube 1A, feeding this knot into the distal end side of the overtube 1A by using a sheath-shaped member, and fastening the knots such that the knots do not shrink while the opening SO is sutured, thereby to suture the opening SO.

In the case that the suture is insufficient, depending on necessity, the suture may be supplemented by introducing other conventionally well-known devices.

After the suturing has been completed, the proximal end side portions of the thread members 34 near the engagement position of the stopper 33B are cut by a cutting instrument introduced from a channel of the endoscope 2, the proximal end sides of the thread members that have been cut are pulled out from the patient, and at the same time, the overtube 1A and the endoscope 2 are removed from the patient. Then, the pressure applied to the stomach ST is released and the manipulation is ended.

According to such a medical procedure, the distal end sides of the thread members 34 are fastened by the anchors 33A to the tissue in proximity to the targeted incision site T, and by pulling the proximal end sides of the thread members 34 during the incision while pressing the tissue in proximity to the targeted incision site T against the cutting electrode 6, which is the cutting instrument, the incision can be made. As a result, while making the incision, the cutting instrument is not displaced from the targeted incision site T, and it is possible to cut the targeted incision site more precisely. At the same time, it is possible to cut such that the cutting instrument reaches the posterior surface of the tissue reliably. In this connection, while making the incision, no tension is applied to the tissue that is to be cut, and if the cutting instrument is simply pressed against the tissue that is to be cut, for example, the tip of the cutting instrument frequently becomes displaced from the targeted incision site T when the surface of the tissue is very slippery or when the site that is to be cut is harder than another site. In the medical procedure of this embodiment, because the tissue in proximity to the targeted incision site T is cut while applying tension using the thread members 34, the tip of the cutting instrument becomes displaced from the targeted incision site T with difficulty.

In addition, because cutting is carried out by applying tension using the thread members 33 only to the tissue that is to be cut, it is possible to form a gap between the stomach wall, which is the tissue that is to be cut, and, for example, the abdominal wall in proximity thereto, and thereby it is possible to cut more easily only the necessary tissue.

In addition, in the overtube 1A of this embodiment, two thread members 34 having the anchors 33A that are attached to the distal end thereof are used, the distal end sides of these thread members 34 are retained by the anchors 33A on both sides of the targeted incision site T so as to situate the targeted incision site T therebetween, and while pulling the proximal end sides of both of these thread members 34, the cutting electrode 6, which is the cutting instrument, is pressed against and cuts the targeted incision site T. Thereby, the displacement of the cutting instrument from the targeted incision site T is restricted on both sides, and it is possible to prevent more completely the displacement of the tip of the cutting instrument from the targeted incision site T.

In addition, because the thread members 34 are fastened in proximity to the tissue that is to be cut and the T-shaped anchors 33A that are attached to the distal ends of the thread members 34 are used, it is possible to fasten the distal ends of the thread members 34 to the tissue easily.

In addition, the hollow puncture needles 32A and 32B are used when the anchors 33A are fastened to the posterior side of the tissue. The anchors 33A are held beforehand in the hollow portion of these puncture needles 32A and 32B, and these puncture needles 32A and 32B penetrate so as to enter from the anterior side and reach the posterior side of the tissue that is to be cut. Subsequently, the anchors 33A are delivered from the hollow portion of these puncture needles. Thereby, the anchors 33A are disposed at the desired location. In addition, by using the hollow puncture needles 32A and 32B in this manner, it is possible to retain the anchors 33A at the desired location on the posterior side of the tissue extremely easily.

In addition, after the prescribed medical procedure in the abdominal cavity AC, by moving the stopper 33B engaged on the proximal end sides of the thread members 33 to the distal end side, it is possible to use the anchors 33A and the thread members 33 that are used while making the incision to suture the opening SO portion that has been cut, and thereby, it is possible to carry out the suturing operation more easily.

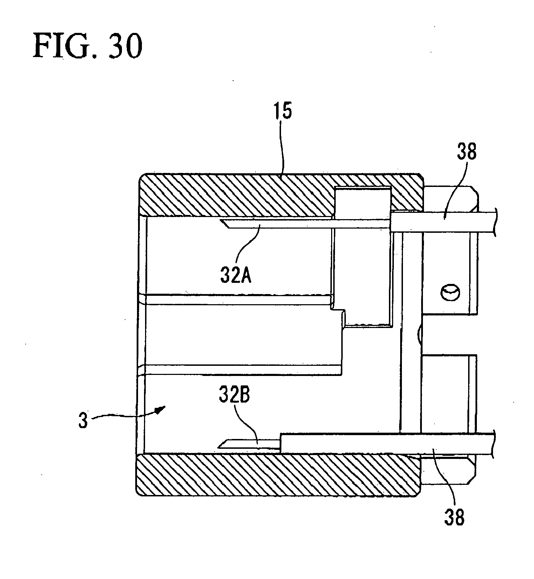

Note that in the embodiment described above, the hollow puncture needles 32A and 32B that are used to retain the anchors are disposed on the outer circumferential surface side of the distal end portion 15 of the overtube 1A, but this is not limiting. As shown in FIG. 30, the hollow, puncture needles 32A and 32B for retaining the anchors may be disposed inside the distal end portion 15 of the overtube 1A (more specifically, the inner circumferential surface that defines the lumen 3). In this case, when the endoscope is disposed inside the overtube 1A, due to this endoscope, it is possible to observe the condition of the tissue that is penetrated by the puncture needles 32A and 32B, and thus, it is possible to retain the anchors at the desired locations more reliably. Third embodiment

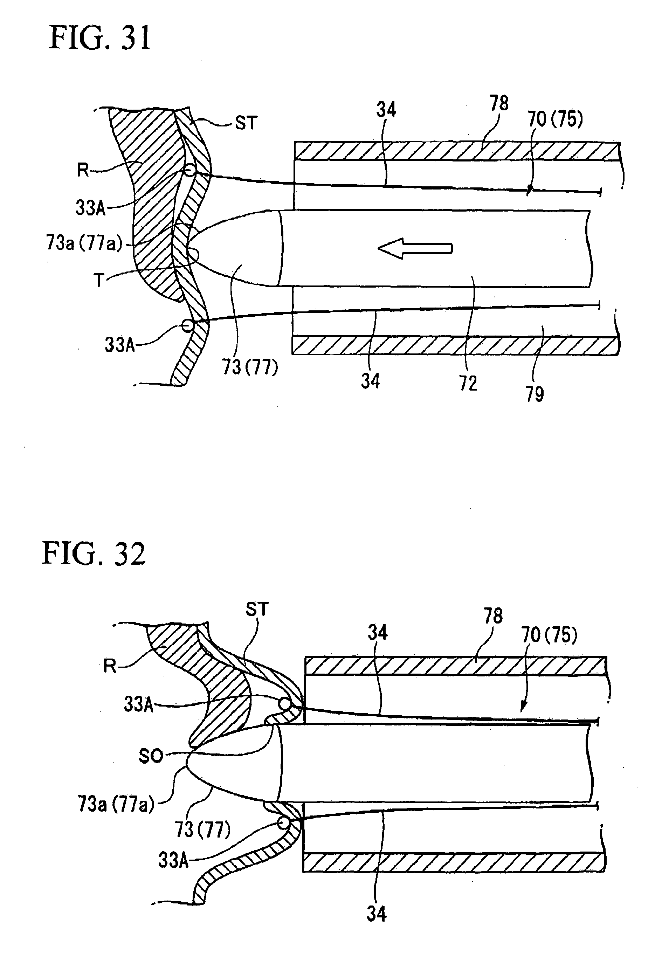

A third embodiment of the present invention will be explained with reference to FIG. 31 to FIG 39.

The third embodiment differs from the second embodiment described above on the point that instead of the cutting electrode 6 using a high frequency, a knife shaped cutting instrument having a tip that can cut tissue on the distal end thereof is used,

Note that in the third embodiment, the cutting step using the knife shaped cutting instrument will be explained, but the other steps, specifically, the insertion step, the inflation step, the guiding step, the suction application step, the insufflation step, the retention step, the treatment step, and the suturing step, are identical to those of the first embodiment described above, and therefore the explanations thereof have been omitted here, This point is also identical with respect to the third and fourth embodiments.

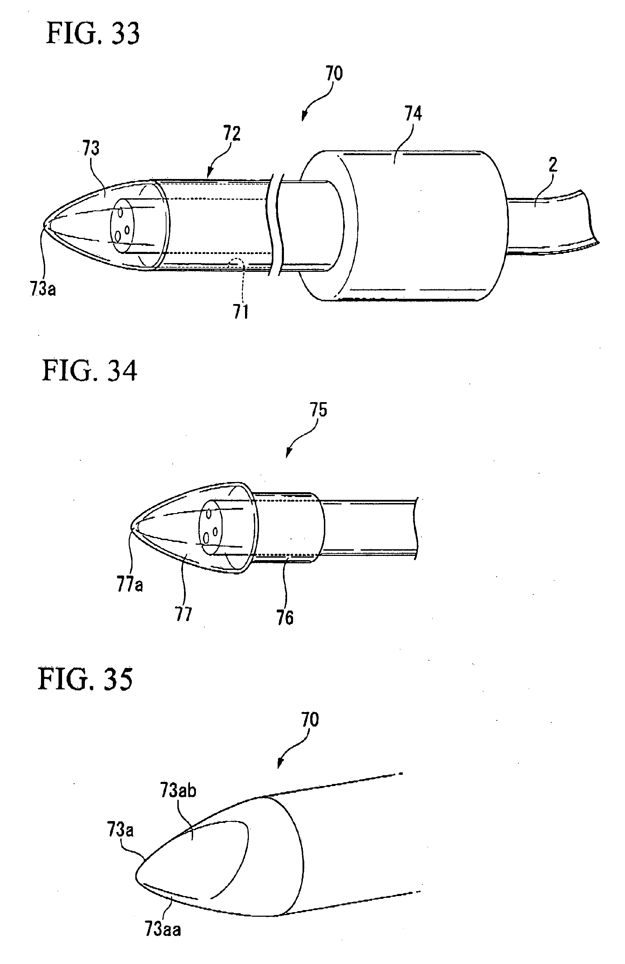

FIGS. 31 and 32 are drawings for explaining the state in which, for example, the stomach wall ST is cut by using a knife shaped cutting instrument 70. As shown in FIG 33, for example, it is possible to use what is called an overtube type cutting instrument as a cutting instrument 70. The overtube-type cutting instrument 70 has a tube shape and is provided with a lumen 71 through which a device such as an endoscope 2 can be passed.

The cutting instrument 70 shown in FIG. 33 is constructed by a tube portion 72, a cutting instrument body 73, and a grip portion 74. The tube portion 72 has an outer diameter that is set smaller than the inner diameter of the overtube 1A so as to be able to be pass through the overtube 1A and has a lumen 71 formed therein. The cutting instrument body 73 is attached to the distal end of the tube portion 72. The grip portion 74 is provided on the proximal end side of the tube portion 72, has a diameter that is larger than the outer diameter of the tube portion 72, and opens in the proximal end side.

The cutting instrument body 73 is made from a transparent material such as glass or an acrylic resin, and on the distal end, a knife tip 73a is formed so as to be able to cut tissue. Note that, depending on necessity, a fastening device that fastens the endoscope 2 that passes through the inner lumen 71 may be provided on the cutting instrument 70 shown in FIG. 33, and thereby, the cutting instrument 70. and the inner endoscope 2 may be integrally constructed and inserted into the overtube 1.

The cutting instrument, as shown in FIG. 33 is not limited to what is called an overtube type. As shown in FIG. 34, what is called an attachment type cutting instrument 75 that is releasably attached to the distal end of the endoscope 2 may be used.

The attachment-type cutting instrument 75 is structured by a fitting portion 76 that fits over the outside of the distal end of the endoscope 2, and a cutting instrument body 77 that is attached to the distal end of the fitting portion 76 and that has a tip 77a. It is not necessary for the entire cutting instrument 75 to be transparent, but preferably at least a portion of the cutting instrument body 77 is made of a transparent material so that the forward visibility of the endoscope 2 to which the cutting instrument 75 is attached can be ensured. A method that can be considered for attaching the cutting instrument 75 to the endoscope 2 is forming the fitting portion 76 of the cutting instrument 75 from an elastic material and fitting the fitting portion 76 onto the distal end of the endoscope 2 by using this elasticity.

The shape of the tips 73a and 77a of the cutting instrument body of the cutting instruments 70 and 75 is not limited to the substantially conical shapes shown in FIGS. 33 and 34, but in addition, the various shapes shown in FIG. 35 to FIG. 39 can be considered. However, while FIG. 35 to FIG. 39 show examples of the tip shape of what is called an overtube-type cutting instrument, of course this is not limiting. These shapes can similarly also be applied to what is called an attachment-type cutting instrument.

FIG 35 to FIG 37 show what is called a knife edge shaped cutting instrument, which has inclined surfaces 73ab respectively on the right and left, the width of these left and right inclined surfaces 73ab gradually narrow toward the distal end, and a flat portion 73aa is formed on the distal end.

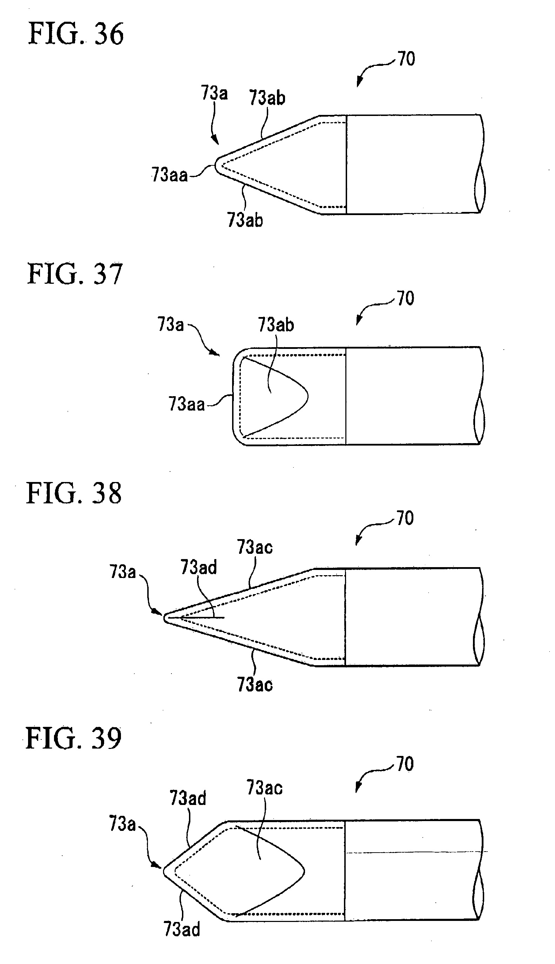

In addition, the cutting instrument shown in FIGS. 38 and 39 has a shape in which there are left and right inclined surfaces 73ac, the width of the left and right inclined surfaces 72ac gradually narrows toward the distal end, and at the same time, when viewing these inclined surfaces 73ac frontally, the distal end tapers to a point similar to an arrow such that the vertical ridge line 73ad is approximately 90 degrees.

Each of the tips 73a shown in FIG. 35 to FIG. 39 forms what is called a dull tip, where the radius of curvature of the distal end thereof is approximately 0.05 mm to 0.5 mm, and this is not particularly sharp. Therefore, this dull tip cannot cut the tissue simply by being pressed against the tissue using a weak force. Unless the dull tip is pressed by a relatively strong force, the tissue cannot be cut.

In the explanation of the medical procedure using this cutting instrument, as described above, by using, for example, the lumen 79 of the overtube 78, the anchors 33A that are attached to the distal ends of the thread members 34 are each retained on the posterior side of the tissue (for example, the stomach wall) that is to be cut on both sides of the targeted incision site T (FIG. 32).

In addition, the endoscope 2 is temporarily removed from the overtube 78, and the overtube type cutting instrument 70 described above is fit onto the outer circumference of the removed endoscope 2. Alternatively, the attachment type cutting instrument 75 described above may be attached to the distal end of the removed endoscope 2.

Then the cutting instrument 70 or the cutting instrument 75 attached in this manner is inserted into the lumen 79 of the overtube 78, and the cutting instrument body 73 (77) on the distal end reaches the vicinity of the targeted incision site T. In this state, the cutting instrument 73 (77) is then pressed farther toward the distal end side. Simultaneously, the proximal end sides of the two thread members 34 that are exposed from the proximal end side opening of the overtube 78 are pulled in the distal direction.

Thereby, as shown in FIG. 31, it is possible to pull the tissue that is present on both sides of the targeted incision site T toward the tip 73a of the cutting instrument body 73 (77) by using each of the anchors 33A that are attached to the distal ends of both thread members 33. As a result, as shown in FIG. 23, the stomach wall ST is cut by the tip 73a (77a) of the cutting instrument body 73 (77), and it is thereby possible to form an opening SO in the stomach wall ST.

Here, the tip 73a (77a) of the cutting instrument body 73 (77) may contact other organs such as the liver R and the abdominal wall which are adjacent to the stomach wall ST. However, because the tip 73a (77a) of the cutting instrument body has a dull shape rather than a sharp one and because tension is not applied to the tissue of the liver R or the like, which are not the objects of the incision by the thread members 33, the tip 73a (77a) of the cutting instrument body does not cut the tissue of the liver R and the abdominal wall or the like, and does not enter into these tissues.

After the incision has been completed, the cutting instrument 70 (77) is again removed from the endoscope 2 and the overtube 1, and the cutting instrument 70 (75) is separated from this removed endoscope 2. Then the endoscope 2, without the cutting instrument attached, is again inserted into the lumen 79 of the overtube 78, and the desired manipulation is carried out in the abdominal cavity AC.

### Fourth embodiment

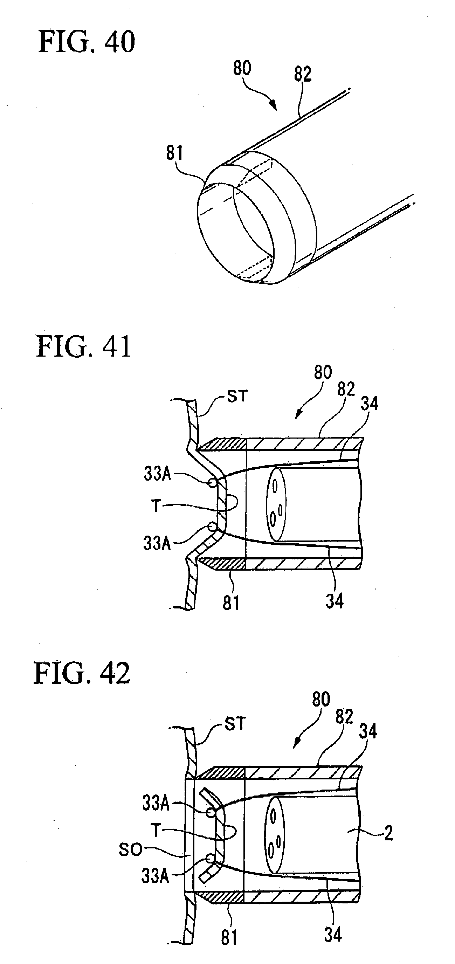

A fourth embodiment of the present invention will be explained with reference to FIG. 40 to FIG. 45.

The fourth embodiment differs from the second embodiment described above on the point that instead of using a high frequency, on the distal ends of the overtubes 80 and 90, tip portions 81 and 91, which are integrally mounted on the overtubes 80 and 90 and can cut tissue, are used as cutting instruments.

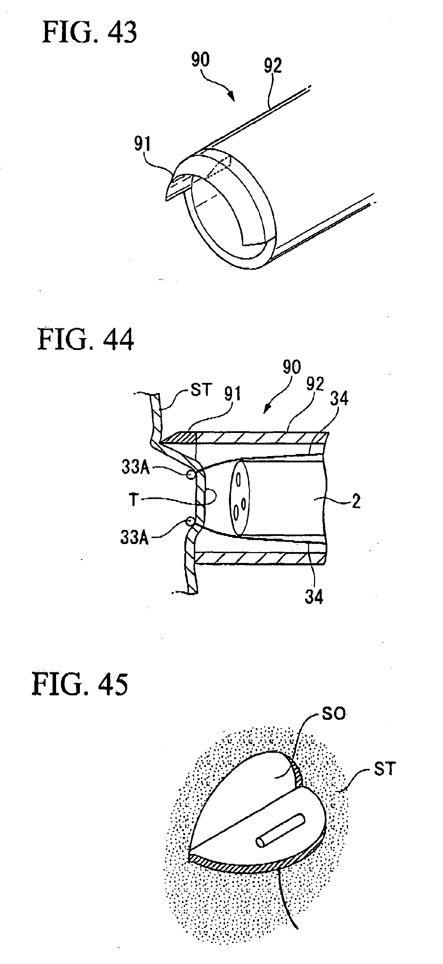

Examples of the tip are, as shown in FIG. 40, the tip portion 81 that is attached along the entire circumference of the distal end of the overtube 80, and as shown in FIG. 43, the tip portion 91 that is only installed on a portion, for example, the upper half, of the distal end of the overtube 90.

The example shown in FIG. 40 is one in which a tip portion 81 made of a metal such as stainless steel is attached to the distal end of the overtube body 82, which is made of a flexible material or a material having a certain degree of rigidity. The tip of this tip portion 81 is preferably what is called a dull tip, in which the radius of curvature of the distal end thereof is comparatively large and not necessarily sharp. This is to prevent damaging tissue that is not the object of cutting.

A medical procedure using this cutting instrument will now be explained. The anchors 33A that are attached to the distal ends of the thread members 34 are retained by establishing in advance a predetermined gap in proximity to the targeted incision site T. The overtube 80 is advanced, and the tip portion 81 that is attached to the distal end thereof reaches the vicinity of the targeted incision site T. In this state, when the overtube 80 is pushed slightly toward the distal end side, simultaneously, the proximal end side of the two thread members 34 that are exposed from the proximal end side opening of the overtube 80 are pulled toward the distal side.

Thereby, as shown in FIG. 41, the tissue that is present at the targeted incision site T can be pulled to the tip portion 81 of the distal end of the overtube by each of the anchors 33A that are attached to the distal ends of both thread members 34. Then, by pulling the thread members 33 with a stronger force, as shown in FIG. 42, the stomach wall ST is cut by the tip portion 81, and it is thereby possible to form a circular opening SO in the stomach wall ST.

After the incision has been completed, using this opening SO portion, the desired medical procedure is carried out by introducing the distal end of the overtube 80 and the insertion portion of the endoscope 2, which is inside the overtube, into the abdominal cavity.

In contrast, as shown in FIG 43, the tip portion 91, which is made, for example, of metal and formed only on the upper half of the overtube 92, is mounted on the distal end of the overtube body 92, which is made of a flexible material or a material having a certain degree of rigidity. The tip of this tip portion 91 is preferably what is called a dull tip, in which the radius of curvature of the distal end thereof is comparatively large and not necessarily sharp. This is to prevent more completely damaging the tissue that is not the object of cutting.

In the overtube 90 using this cutting instrument, the anchors 33A that are attached to the distal ends of the thread members 34 are retained on the posterior side of the tissue (for example, the stomach wall) that is to be cut and a predetermined gap in proximity to the targeted incision site T is established in advance. Then the overtube 90 that has reached the vicinity of the targeted incision site T is pressed slightly toward the distal end side, and simultaneously, the two thread members 34 that are exposed from the proximal end side opening of the overtube 90 are pulled toward the distal side. Thereby, as shown in FIG. 44, the tissue that is present at the targeted incision site T can be pulled to the tip portion 91 of the distal end of the overtube by the anchors 33A that are attached to the distal ends of both thread members 34. By pulling the thread members 34 with a stronger force, as shown in FIG. 45, the stomach wall ST is cut by the tip portion 91, and it is thereby possible to form a semi-circular opening SO in the stomach wall ST.

Note that an overtube into which the overtube 80 shown in FIG 40 to FIG 45 is inserted, for example, an overtube having a structure in which the cutting electrode is eliminated from the overtube shown in the first embodiment, may also be used as a device into which the cutting instrument (overtube 80) is inserted. In this case, it is possible to make the tip portion 81 sharp.

### Fifth embodiment

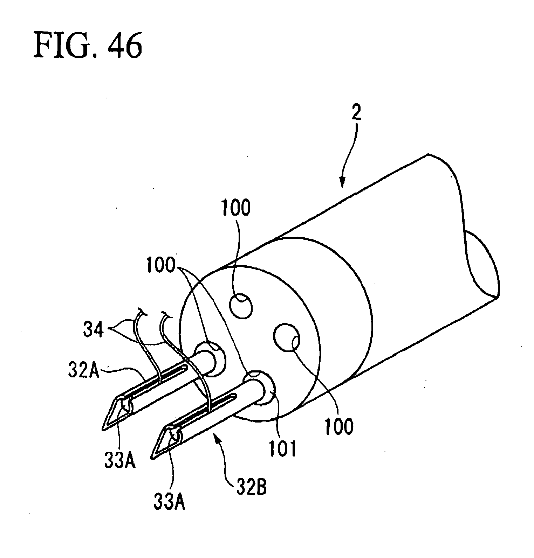

A fifth embodiment of the present invention will be explained with reference to FIG. 46 to FIG. 48.

The fifth embodiment differs from the second embodiment described above on the point that the anchors of the thread members 34 are retained on the posterior side of the tissue by using a channel 100 of the endoscope 2 instead of the overtube, and the point that the proximal end sides of the thread members 34 are pulled outside the body of the patient directly, without using the lumen of the overtube.

Specifically, in this embodiment; the outside sheath 101 having hollow puncture needles 32A and 32B in the distal end thereof is inserted in advance into a channel 100 of the endoscope 2. Then each of the anchors 33A is set by being held in the hollow puncture needles 32A and 32B, and at the same time, the thread members 34 that extend from the anchors 33A are passed through the outside sheath 101 or through another channel 100 of the endoscope.

The endoscope 2 in which the outer sheath 101 or the like has been set in advance is inserted into the stomach (a luminal organ) through the mouth of a patient. Then the insertion portion of the endoscope 2 is brought into proximity to the targeted incision site. After the targeted incision site has been confirmed, the needle controlling portion is operated (not illustrated), the puncture needle 32A is projected from the distal end of the endoscope 2, and the tissue in proximity to the targeted incision site is punctured. In this state, a pusher control portion (not illustrated) is operated, and the anchors 33A are delivered by the pushers from the hollow portion of the puncture needle 32A and retained on the posterior side of the tissue. In contrast, a similar operation is carried out with the puncture needle 32B, which has been set in the other channel, and the anchor 33A is delivered to and retained on the posterior side of the tissue on the other side such that the targeted incision site is situated therebetween.

Next, the endoscope that has delivered the anchors 33A is temporarily removed through the mouth of the patient, and the overtube type cutting instrument 70 explained in the second embodiment is fit on the outer circumference of this extracted endoscope 2 or the attachment type cutting instrument 75 is attached to the distal end of the endoscope 2.

In addition, this cutting instrument 70 or the cutting instrument 75 is again inserted integrally with the endoscope 2 into the stomach (a luminal organ) ST through the mouth of the patient. Then the cutting instrument 70 (75) at the distal end of the endoscope reaches the vicinity of the targeted incision site T, and in this state, the cutting instrument 70 (75) is pushed farther in the distal end direction. Simultaneously, the proximal end sides of the two thread members 33, which are exposed from the proximal end side opening of the overtube 1A, are pulled distally.

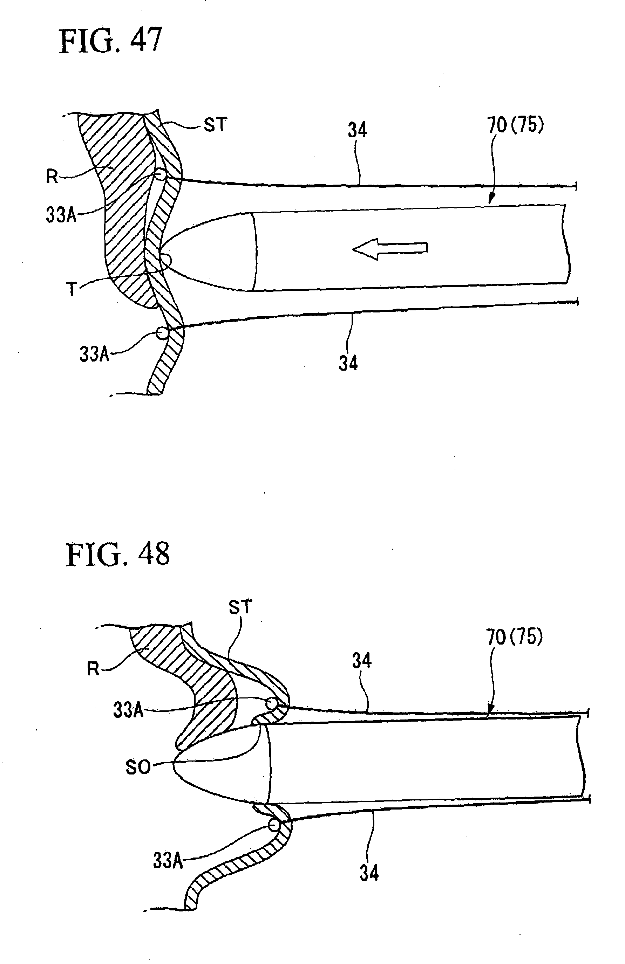

Thereby, as shown in FIG 47, the tissue that is present on both sides of the targeted incision site T can be pulled to the tip of the cutting instrument body 73 (77) by each of the anchors 33A that are attached to the distal ends of both thread members 33. Then by pulling the thread members 33 with a stronger force, as shown in FIG. 48, the stomach wall ST is cut by the tip of the cutting instrument body 73 (77), and thereby it is possible to form an opening SO in the stomach wall ST.

### Sixth embodiment

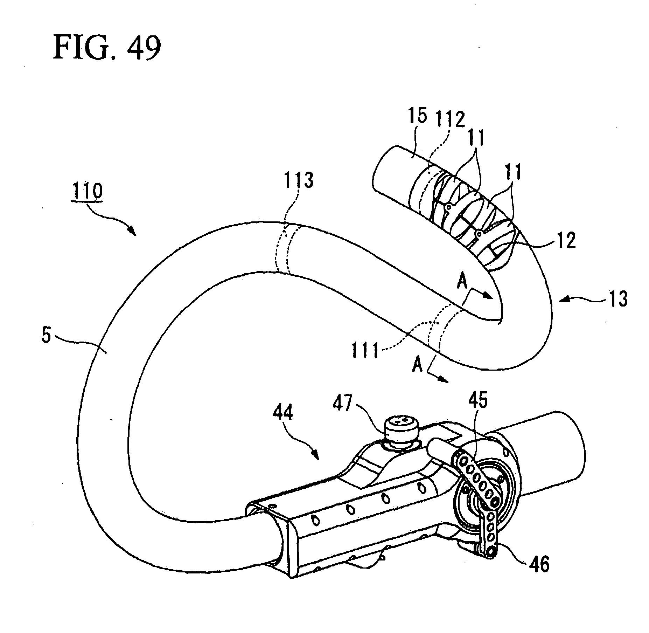

A sixth embodiment according to this invention shall now be described with reference to FIGS. 49 to 57.

A point of difference of the sixth embodiment with respect to the first embodiment is that an overtube 110 according to this embodiment has a first magnet (magnetic body) 111, disposed on an outer peripheral surface of the insertion part 5 near the proximal end of the bending part 13, a second magnet (magnetic body) 112, disposed on an outer peripheral surface at the distal end of the bending part 13, and a third magnet (magnetic body) 113, disposed more toward the proximal end side (manipulating handle 21 side) of the insertion part 5 than the first magnet 111, as shown in FIG. 49.

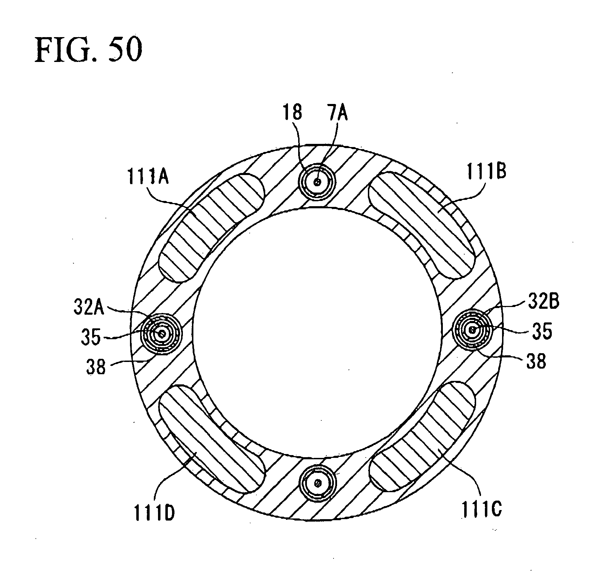

In order to restrain the insertion part 5 of the overtube 110 from becoming large in diameter and yet secure the inner diameter of the lumen 3, the first magnet 111 (and likewise, the second magnet 112 and the third magnet 113) is, for example, divided into and disposed as magnet pieces 111A, 111B, 111C, and 111D at portions besides portions at which the electrode tube 18 and the outer sheaths 38, inserted through the insertion part 5, are disposed as shown in FIG. 50.

Each of these magnets 111, 112, and 113 is formed so that all of the outer peripheral surface is of the same magnetic pole and these magnets are arranged so that the magnetic poles alternate along the insertion part 5, for example in a manner such that when the first magnet 111 is of the S pole, the second magnet 112 and the third magnet 113 are of the N pole.

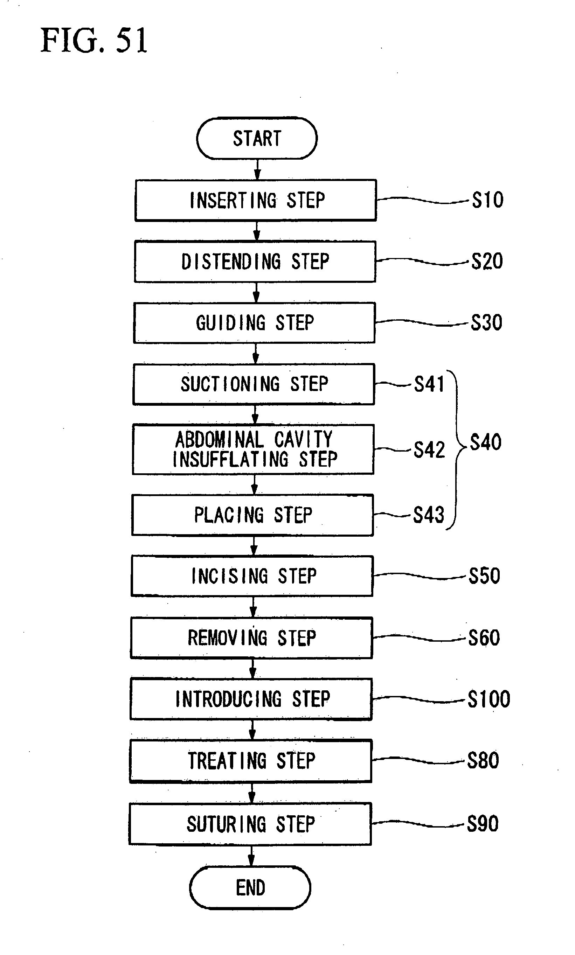

Actions of this embodiment shall now be described in line with a medical procedure performed via a natural orifice using the overtube 110 as shown by the flow chart of FIG. 51.

As in the first embodiment, the steps from the inserting step (S10) to the removing step (S60) are carried out in this embodiment as well.

An introducing step (S100) is then performed. That is, as shown in FIG. 19, the endoscope inserting part 51 of the endoscope 2 is introduced inside the abdominal cavity AC through the opening SO.

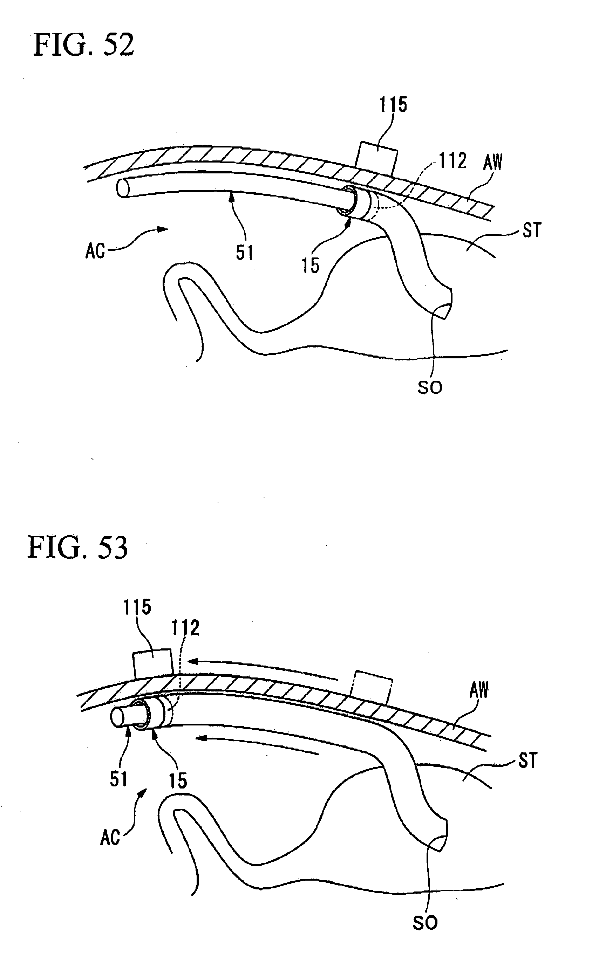

Then with the distal end part 15 of the overtube 110 being protruded from the opening SO of the stomach ST, a moving magnet 115 is placed on an abdominal wall AW near the opening SO with the magnetic pole that is attracted to the second magnet 112 of the overtube 110 being set at the inner side as shown in FIG. 52. In this process, the moving magnet 115 and the second magnet 112 are attracted to each other. The moving magnet 115 is then moved along the abdominal wall AW to a position at which a treatment site is located. In this process, the distal end 15 moves while being attracted to the moving magnet 115.

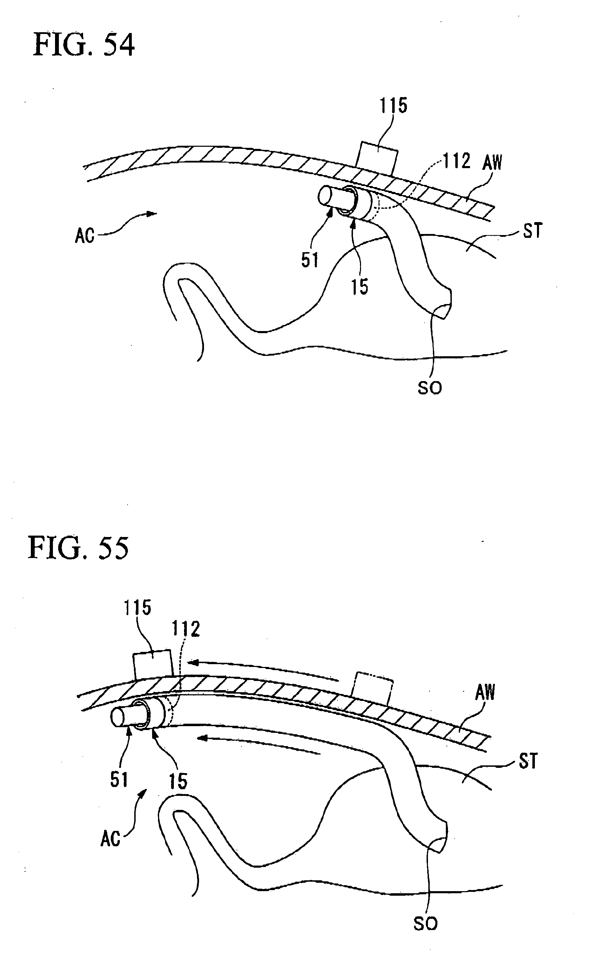

The endoscope inserting part 51 may be advanced with respect to the insertion part of the overtube 110 in advance as shown in FIG. 52, and then the moving magnet 115 may be used to advance the distal end part 15 of the overtube 110 along the endoscope inserting part 51 toward the distal end of the endoscope inserting part 51 as shown in FIG. 53. Or, the moving magnet 115 may be moved with the endoscope inserting part 51 being accommodated inside the overtube 110 as shown in FIG. 54, and then the endoscope inserting part 51 may be moved along with the overtube 110 as shown in FIG. 55.

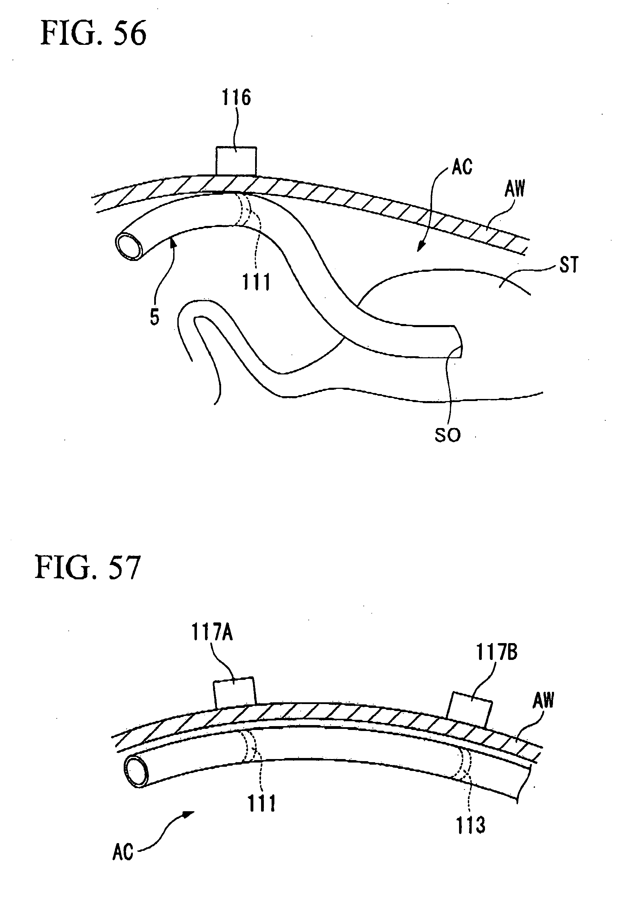

In order to secure a bended state of the bending part 13, a fixing magnet 116 is placed on the abdominal wall AW with the magnetic pole attracted to the first magnet 111 being set at the inner side as shown in FIG. 56. Since the fixing magnet 116 and the first magnet 111 are attracted to each other, a bending manipulation is performed by manipulating the bending lever 45 with the insertion part 5 being fixed to and supported on the abdominal wall AW. Here, in order to change the direction of the distal end part 15 with the bended state of the inserted part 5 being maintained, supporting magnets 117A and 117B are placed on the abdominal wall AW. That is, the supporting magnet 117A and the first magnet 111 are made to be attracted to each other, and the supporting magnet 117B and the third magnet 113 are made to be attracted to each other, When the mutual attraction state is realized, for example, the first magnet 111 side is rotatingly moved about the third magnet 113 side with the third magnet 113 side being fixed to change the direction of the distal end part 15 as shown in FIG. 57.

After then carrying out the treating step (S80), the endoscope 2 is returned into the stomach ST from the opening SO of the stomach wall and taken out from the mouth M of the patient PT, and then the suturing step (S90) is performed. The opening SO of the stomach wall is then sutured.

After suturing, the endoscope 2 is drawn out of the patient, the pressure applied to the abdominal cavity AC is released, and the surgical procedure is ended.

With the overtube 110, the same actions and effects as those of the first embodiment can be exhibited, In particular, since the first magnet 111, the second magnet 112, and the third magnet 113 are disposed at the outer portions of the insertion part 5, these can be attracted to the moving magnet 115, the fixing magnet 116, and the supporting magnets 117A and 117B to thereby support the insertion part 5 on the abdominal wall AW. The endoscope 2 that has been inserted into the overtube 110 can thus be restrained preferably from moving away during treatment. Also, by movement of the moving magnet 115, the distal end direction of the endoscope 2 that has been inserted into the overtube 110 can be moved readily and the direction of endoscope 2 can be controlled readily by the magnets. Also, movement, fixing, and supporting of the overtube 110 can be performed from outside the body by using the moving magnet 115, the fixing magnet 116, and the supporting magnets 117A and 117B to further facilitate orientation of the overtube 110.

### Seventh embodiment

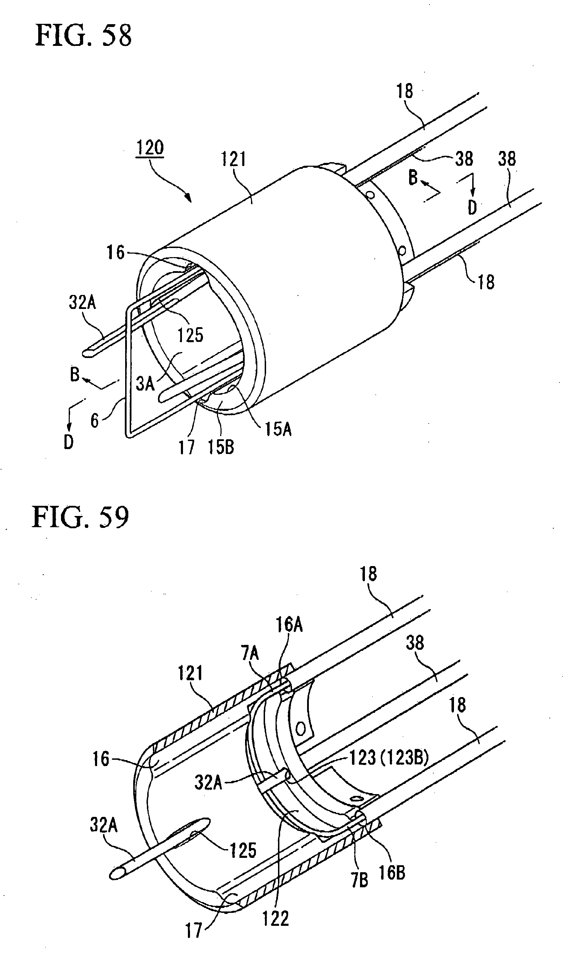

A seventh embodiment according to this invention shall now be described with reference to FIGS. 58 to 67.

The seventh embodiment differs from the first embodiment described above on the point that puncture needles 32A and 32B of the overtube 120 of this embodiment act as a holding portion that holds the tissue cutting electrode 6 at a position that is removed from a position traversing a first lumen 3 while the lumen 3 of the overtube 1 of the first embodiment is used as the first lumen 3.

The cutting electrode 6 is a wire made, for example, of stainless steel and Capable of high frequency conduction. During an incision in which the tissue is cut (first state), the cutting electrode 6 is disposed so as to traverse the center portion of the first lumen 3 in a direction perpendicular to the axial direction of the insertion portion 5. The length of the cutting electrode 6 is formed so as to be longer than the inner diameter of the first lumen 3. That is, as shown in the state of use in FIG. 59, one end of the cutting electrode 6 is disposed in the first inner groove 16 that is formed in the outer edge of the first lumen 3 (in other words, the inner peripheral surface or inside of the distal portion 121 that defines the first lumen 3), which is the inner surface of the distal end portion 121. The other end of the cutting electrode 6 is disposed in the second inner groove 17 formed in the outer edge (in other words, the inner peripheral surface or inside of the distal end portion 121 that defines the first lumen 3) at a substantially symmetrical position with respect to the first inner groove 16 across the center of the first lumen 3.

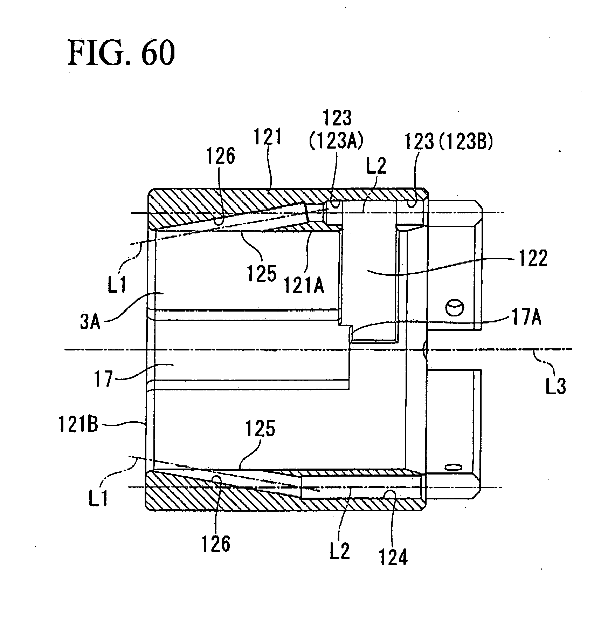

In the accommodation state (second state) shown in FIG. 59, the cutting electrode 6 is accommodated in the accommodating portion 122 formed on the inner peripheral surface of the distal end 121. The accommodating portion 122 is a groove that is provided from the first inner groove 16 to the second inner groove 17 along the peripheral direction as shown in FIGS 59 and 60. The end portion of the accommodating portion 122 is disposed slightly closer to the distal end than the steps 16A and 17A that abut the electrode tubes 18.

As shown in FIG. 60, on the inner peripheral surface 121A of the distal end 121 of the overtube 120, long holes 125 are each formed having a long axis in the longitudinal direction (the direction that joins the distal end and the proximal end of the insertion portion 5) of the distal end portion 121 in a position substantially perpendicular to the direction in which the first inner groove 16 and the second inner groove 17 are connected. The long holes 125 serve as the distal end opening (the opening from which the puncture needles 32A and 32B project) of the needle lumens 123 and 124 through which the puncture needles 32A and 32B pass. In this embodiment, the center of the long holes 12S is provided more toward the proximal end side than the distal end opening 3A of the distal end portion 121.

The long holes 125 communicate with the guide portions 126 that form the lumens through which the puncture needles 32A and 32B pass, and the guide portions 126 are formed so as to incline inside the distal end portion 121. The proximal end sides of the each of the guide portions 126 form conduits that communicate with the distal ends of each of the needle lumens 123 and 124 formed on the proximal end portion of the distal end portion 121, thereby forming a second lumen along with the needle lumens 123 and 124.

The center axes of the needle lumens 123 and 124 are formed substantially parallel to the axis L3 (also referred to as the center axis) of the first lumen 3, are provided at opposing positions across the approximate center of the first lumen 3, and are formed mutually parallel in their longitudinal direction. The axes (center axes) L1 of guide portions 126 are inclined inward (on the axis of the first lumen (or on the center axis of the first lumen 3)) at an angle of 5 º to 15 º with respect to the axis of the needle lumens 123 and 024. Note that the needle lumen 123 is provided at a location that passes through the accommodating portion 122 for the cutting electrode 6 described above.

Therefore, the needle lumen 123 is partitioned into a portion 123A more toward the distal end side than the accommodating portion 122 and a portion 123B that is more toward the proximal end side than the accommodating portion 122. The external sheaths 38 pass through the portion 123B and the needle lumen 124. The distal end of the external sheaths 38 respectively abut the distal end portion of the needle lumen 124 and the distal end portion of the portion 123B, and are thereby connected and fastened.

Next, actions of the overtube 120 according to this embodiment shall now be described as in the first embodiment with a natural orifice transluminal medical procedure using the overtube 120.

First, as a preparatory step, the electrode controlling wires 7A and 7B are passed through the electrode tubes 18, and the cutting electrode 6 is disposed at the distal end portion 121. A jig (not illustrated) is inserted into the distal end opening 3A of the distal end portion 121 and the cutting electrode 6 pressed and inserted into the accommodation portion 122. In this state, the puncture needles 32A and 32B are passed through the external sheath 38. As shown in FIG. 59, the puncture needle 32A traverses the accommodation portion 122, and the cutting electrode 6 is disposed between the puncture needle 32A and the inner peripheral wall of the accommodation portion 122. The puncture needle 32A that traverses the accommodation portion 122 serves as an holding portion, and thereby the cutting electrode 6 is inhibited from projecting into the first lumen 3. When this action has finished, the jig (not illustrated) is extracted.

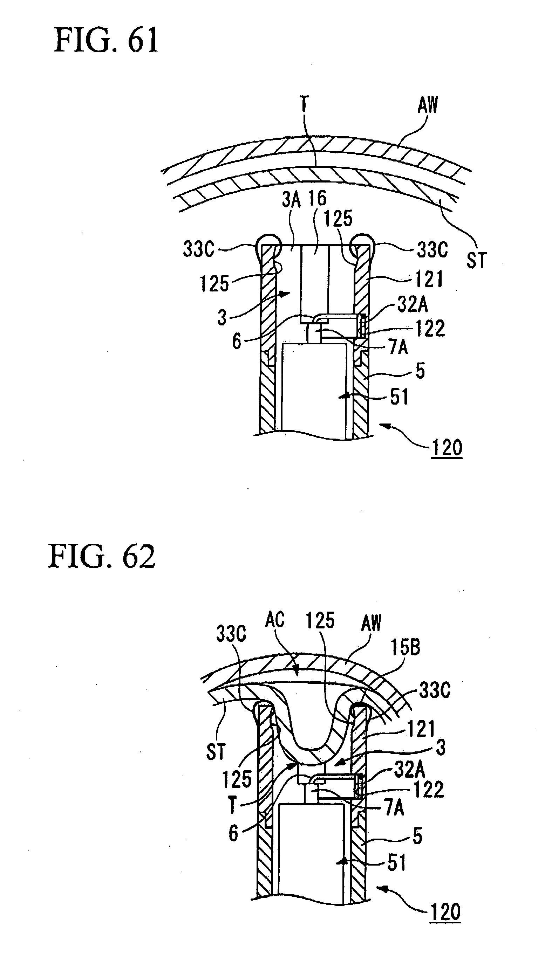

When the medical procedure is carried out, an insertion step (S10), an insufflation step (S20), and a guidance step (S30) are carried out as in the first embodiment, and then it becomes the state shown in FIG. 61

Then the process moves to the needle moving step (S40), in which the puncture needles 32A and 32B disposed in the distal end side of the insertion portion 5 are advanced and retracted along the first lumen 3. First, as a suction step (S41), while the distal end portion 121 is abutted against the stomach, including the targeted incision cite T, the stomach wall is sucked by the suction apparatus 63 via the channel 58. Note that in the case that the distal end of the endoscope insertion portion 51 (distal end of the device) projects relative to the overtube 120 (i.e., the case in which the distal end of the endoscope insertion portion 51 that has passed through the first lumen 3 projects more than the distal end of the overtube 120), the distal end of the endoscope insertion portion 51 is drawn back such that the distal end of the endoscope insertion portion 51 is disposed in the first lumen 3.

Thereby, as shown in FIG. 62, a portion of the stomach wall is drawn into the first lumen 3 of the distal end portion 121 from the distal end opening 3A. Thereby, a space is established between the outside of the stomach wall and the abdominal cavity AC. Here, as a means for sucking the stomach wall, the method of using the channel 58 of the endoscope 2 is not limiting. For example, the space formed between the inner surface of the first lumen 3 of the overtube 120 and the outer periphery of the insertion portion of the device such as the endoscope 2 that has been inserted in the first lumen 3 may be used as a suction passage, and suction may be implemented by connecting the suction passage to a suction apparatus 63. In this case, the suction can be further improved by providing a valve (not illustrated) in the established space that inhibits the flow of fluids between the inside and outside of the body.

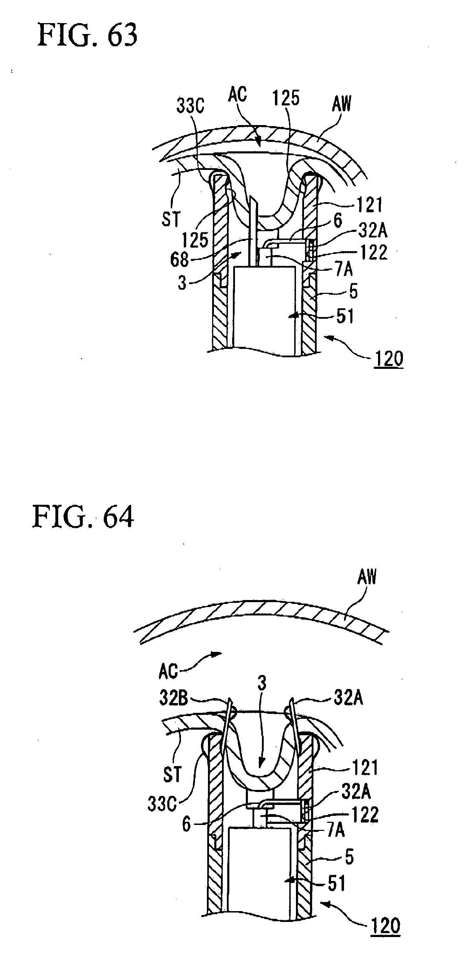

Next, the process proceeds to a peritoneum insufflation step (S42). First, the peritoneum insufflation needle 68, which is connected to the air and water supplying apparatus 62, is inserted into the channel 60 of the endoscope 120. Then the distal end of the peritoneum insufflation needle 68 is projected into the distal end portion 121, and as shown in FIG 63, inserted farther up to the abdominal cavity AC by puncturing the sucked stomach wall. Thereby, while the space between the sucked stomach wall and the abdominal wall AW is established, the stomach wall is punctured by the peritoneum insufflation needle 68, and thereby it is possible to puncture only the stomach wall reliably. Next, air is passed into the abdominal cavity AC via the peritoneum insufflation needle 68 and the abdominal cavity AC is inflated so that the stomach ST and the abdominal wall AW are separated.

Next, the process proceeds to a retention step (S43). Here, first the sheath grip portion 40 is grasped, and the needle control handle 41 is advanced in the direction of the sheath grip portion 40. As shown in FIG. 64, the puncture needles 32A and 32B advance toward the inside (the center of the first lumen 3) along the guiding portion 126, project from the long holes 30, and puncture the portion of the stomach wall that is being sucked. Because a space has been established between the abdominal cavity AC and the stomach wall by insufflation, the puncture needles 32A and 32B puncture only the stomach wall. In addition, the puncture needles 32A and 32B enter the stomach wall toward the inside following the inclination of the guiding portion 126, and thus it is possible to confirm visually the movement of the puncture needles 32A and 32B, the entrance position, and the definite entry of the puncture needles 32A and 32B into the stomach wall by using the observation apparatus 55 in the endoscope 2. In addition, because the distal portion 121 is transparent, the movement of the puncture needles 32A and 32B inside the needle lumens 123 and 124 can be easily observed.

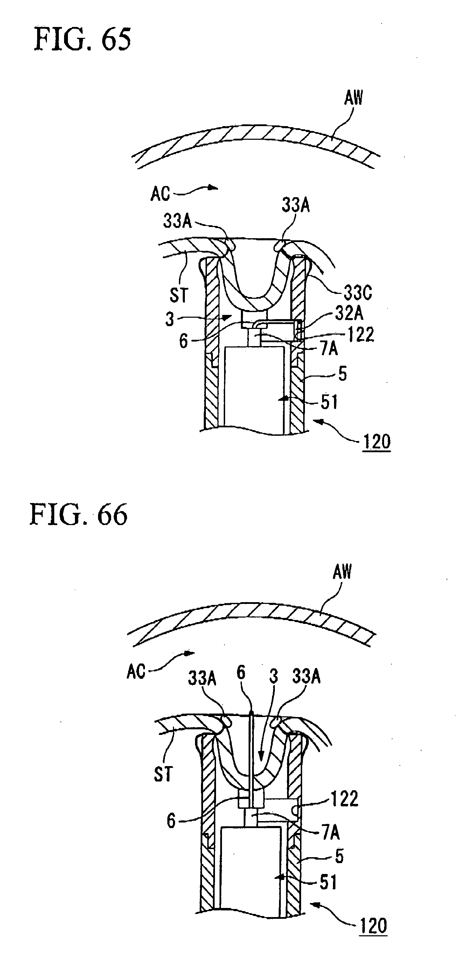

From this state, the pusher connecting portion 43 is advanced towards the needle control handle 41, and the pushers 35 are moved in the direction of the distal end of the puncture needles 32A and 32B. At this time, as shown in FIG 65, the anchors 33A of the double T bar 33 are pushed by the pushers 35 and delivered from inside the puncture needles 32A and 32B into the abdominal cavity AC (the exit side of the punctured tissue). Here, because the hole 37 is oriented from the proximal end side toward the distal end side of the insertion portion 5, the stopper 33B of the double T bar 33 is inhibited from falling out when not intended. At this time, because the abdominal cavity AC has been insufflated and the space between the abdominal cavity AC and the stomach wall has been established, it is possible to puncture only the stomach wall.

After the anchors 33A of the double T bar 33 are released, the pusher connecting portion 43 is retracted towards the needle control handle 41 to accommodate the pushers 35. Then the puncture needles 32A and 32B are retracted and withdrawn from the stomach wall. At this time, the two anchors 33A of the double T bar 33 are opened in a T-shape due to the bending of the sutures 33C.

Next, the process proceeds to the incision step (S50). First, after the distal end of the endoscope 2 is disposed farther toward the proximal side than the accommodating portion 122, the sheath grip portion 40 is grasped, pulled toward the proximal side, and thereby the puncture needles 32A and 32B are extracted from the distal end portion 121 (or the overtube 120). Because the engagement of the cutting electrode 6 by the puncture needle 32A is released, the cutting electrode 6 is restored so as to traverse the first lumen 3 due to the elastic force thereof. Note that the shape of the cutting electrode 6 may also be restored by moving the electrode controlling wires 7A and 7B forward and backward. This action may also be carried out before the stomach wall suction. Because a space is established that is wider than the distal end portion 15 before the stomach wall suction, there is the effect that the cutting electrode 6 can be easily restored to the center.

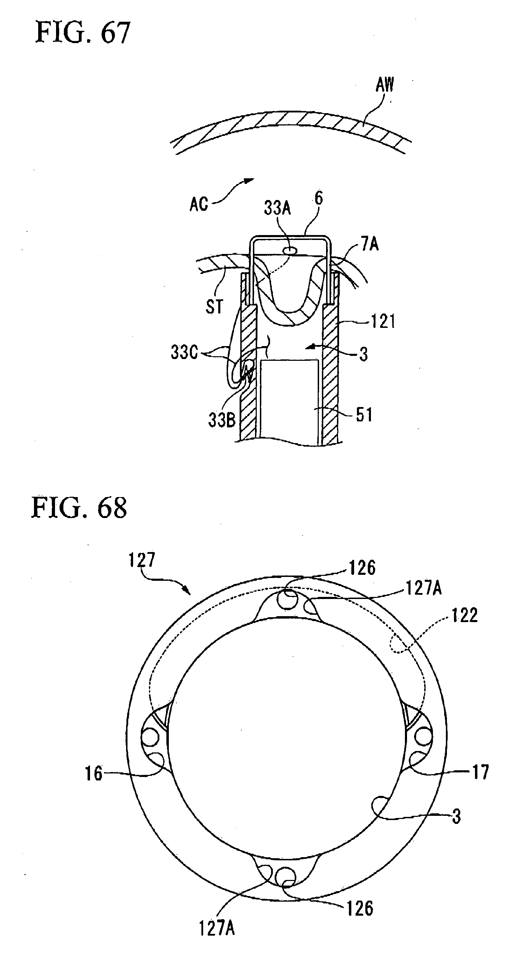

The connection between the connecting terminal 26B of the power cord 28 and the connecting terminal 26A of the electrode controlling portion 8 is confirmed. Then, while supplying high frequency power from the high frequency power source 27, the control handle 21 is advanced toward the control body portion 20, and the cutting electrode 6 is projected from the distal end portion 121 to abut the stomach wall. At this time, because the cutting electrode 6 is energized via the electrode controlling wires 7A and 7B, as shown in FIGS. 66 and 67, the stomach wall is cut open by the cutting electrode 6, and an opening SO is formed in the stomach wall. Note that in this step as well, the suction on the stomach wall is continued, and thus the retention position of the double T bar 33 and the incision position are in an optimal state.

Next, the process proceeds to a removal step (S60), an introduction step (S70), a treatment step (S80), and a suture step (S90) as in the first embodiment. After suturing, the endoscope 2 is removed from the patient, the pressure applied to the abdominal cavity AC is released, and the manipulation is completed.

According to this overtube 120, because an accommodation portion 122 is provided and the cutting electrode 6 is accommodated so as not to project into the first lumen 3, the advancement and retraction of the device (in this embodiment, the endoscope 2) and the visual field of the observation apparatus provided on the device can be ensured. Furthermore, because it is possible to project the device from the distal end of the overtube 120, the character of insertion into the body can be further improved.

At this time, the puncture needle 32A is used as a holding member to engage and hold the cutting electrode 6 that is disposed in the accommodation portion 122. Thereby, it is possible to remove the cutting electrode 6 reliably from the path in which the device is advanced and retracted in the first lumen 3 without providing a separate member. Furthermore, by inserting and removing the puncture needle 32A, it is possible to switch between the accommodation state and the use state of the cutting electrode 6.

Thereby, when the device such as the endoscope 2 is advanced and retracted relative to the overtube 120, the cutting electrode 6 is prevented from becoming an obstacle.

Because the electrode controlling wires 7A and 7B can be separated from the electrode controlling portion 8, the cutting electrode 6 can be removed from the insertion portion 5 along with the electrode controlling wires 7A and 7B. Therefore, after forming the opening SO, when the endoscope 2 projects from the first lumen 3, the cutting electrode 6 is not an obstruction, and when the endoscope 2 passes through the first lumen 3, the endoscope 2 can be advanced farther into the abdominal cavity AC past the tissue that has been cut open. Furthermore, after forming the opening by cutting the tissue, when the device (in this embodiment, the endoscope 2) that has passed through the first lumen 3 advances to project from the distal end of the overtube 120, the operation of retracting the cutting electrode 6 from the passage of the device and the operation of removing the cutting electrode 6 by temporarily removing the overtube 120 from the body can be omitted. As a result, it is possible to reduce the time for the manipulation up to introducing the endoscope 2 into the abdominal cavity AC after opening the stomach wall.

In addition, it is possible to retain the anchors 33A by engaging the sutures 33C before forming the opening by cutting the wall (in this embodiment, the stomach wall) of the abdominal organs. Here, because the long hole 30 (the opening through which the puncture needles 32A and 32B project) is disposed inside the distal end portion, the operating conditions of the puncture needles 32A and 32B can be more easily confirmed by the observation apparatus provided on the device. In addition, because the projection direction of the puncture needles 32A and 32B is controlled by projecting the puncture needles 32A and 32B along the guide portions 126 from the long holes 125 toward the front of the first lumen 3 in the axial (center axis) L3 direction, it is possible to insert the puncture needles 32A and 32B into the tissue while a space is established between the inner surface of the distal end portion 121 and the position at which the needles 32A and 32B puncture the tissue.

In other words, it is possible to set the entrance position of the puncture needles 32A and 32B to a portion (farther inside than the abutting position 1218 of the distal end portion 121) separated from the end portion of the tissue that is held inside the first lumen 3 by suction. Thus, when the puncture needles 32A and 32B are inserted, the tissue (in this embodiment, the stomach wall) is not easily detached.

Furthermore, because the anchors 33A are retained and the sutures 33C pass through the stomach wall before forming the opening (before suturing), when the targeted incision site T is cut open by the cutting electrode 6, movement of the tissue is prevented, and thereby cutting can be carried out more reliably. In addition, this is possible in a state in which the sutures 33C are only bound during suturing. When the opening is sutured after the medical procedure in the abdominal cavity AC has been completed, it is possible to carry out the suturing of the opening more easily without insufflating the stomach, and thereby the suturing operation becomes easier. Here, when the puncture needles 32A and 32B are not used, it is possible to accommodate the puncture needles 32A and 32B in the needle lumens 92 and 93 formed in the distal end portion 15, and thus the puncture needles 32A and 32B do not interfere when a device such as the endoscope 2 are being operated.

In addition, because the direction in which the cutting electrode 6 traverses the first lumen 3 is perpendicular to the direction at which the puncture needles 32A and 32B are joined to the cutting electrode 6, it is possible to separate the puncture position of the puncture needles 32A and 32B from the incision location.

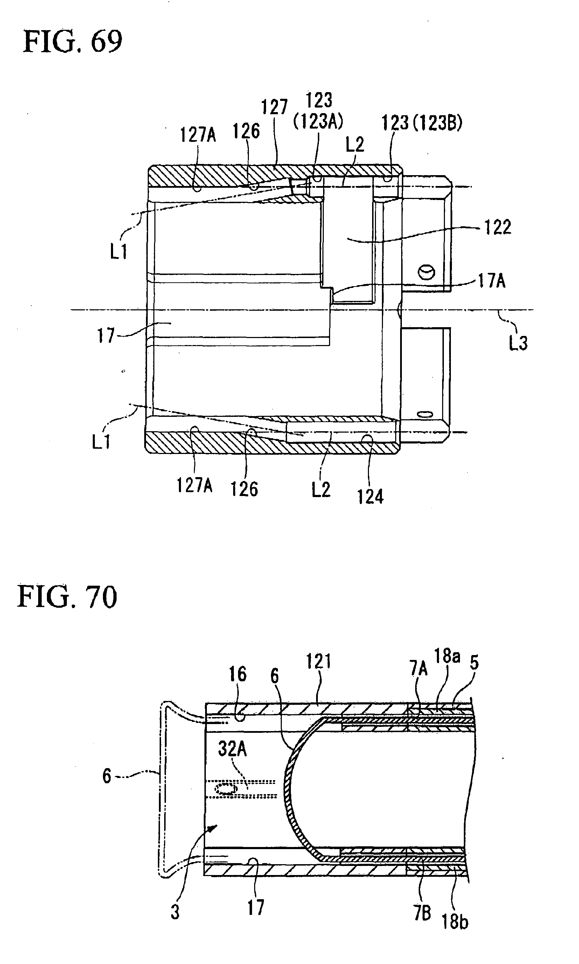

Here, modified examples are shown in FIGS. 68 and 69. A notched portion 127A is formed in the distal portion 127 by cutting a portion of the inside periphery that includes the guide portion 126. The notched portion 127A is formed only in the vicinity of the guide portion 126, but may extend up to the vicinity of the inner grooves 16 and 17. Due to the notched portion 127A, a larger space can be established the around of the puncture needles 32A and 32B, or in other words, the space between the entry position of the puncture needles 32A and 32B and the inner surface of the distal end portion 127. Because it is possible to suck more tissue in the region around the puncture needles 32A and 32B, there is the advantage that the tissue does not easily detach during puncture.

Note that in this embodiment, a structure and method were explained in which the cutting electrode 6 is accommodated in the accommodation portion 122 by being held back by the puncture needles 32A and 32B. However, in the case that there is a sufficient gap between the insertion portion 5 and the endoscope 2, the cutting electrode 6 may be disposed between the insertion portion 5 and the endoscope 2 without being accommodated in the accommodating portion 90, and thus the endoscope 2 may project from the insertion portion 5 to improve the characteristics of the insertion into the body.

At this time, because there is a possibility that the cutting electrode 6 may be pressed by the endoscope 2 and have a bend imparted thereto when the endoscope 2 is moved relative to the insertion portion 5, the cutting electrode 6 is accommodated and protected in the electrode tubes 18. Instead, the electrode controlling wires 7A and 7B may be exposed and the cutting electrode 6 exposed from the electrode tubes 18 and used only when cutting tissue (the stomach wall).

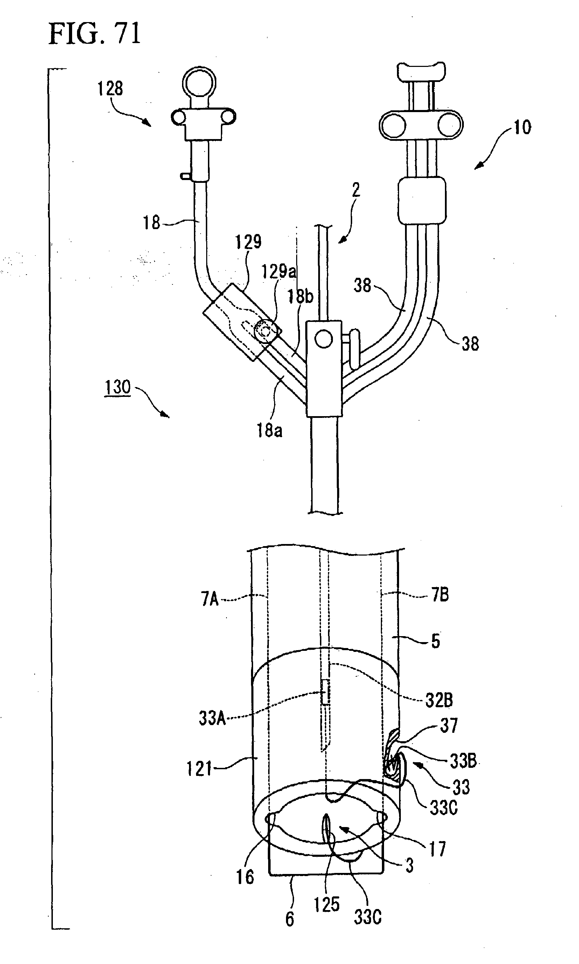

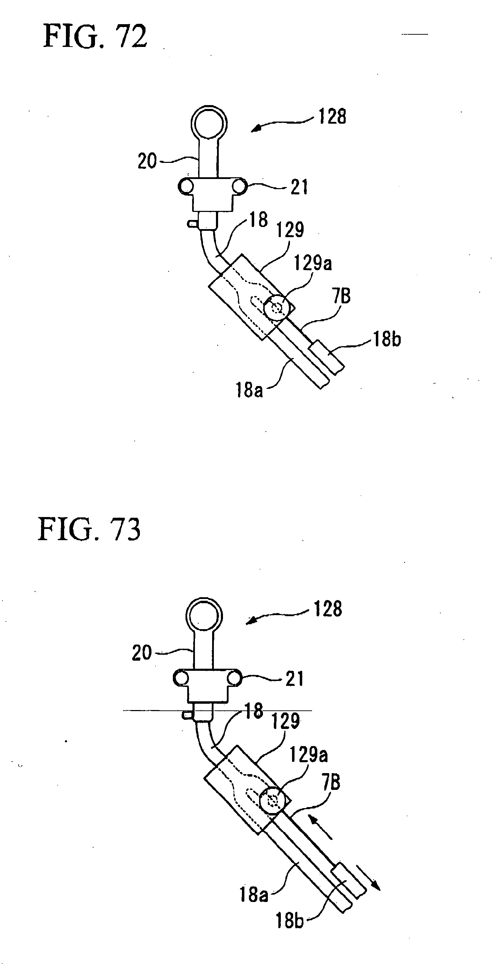

As a method of accommodating the cutting electrode 6 in the electrode tube 18 and exposing the cutting electrode 6 during cutting, as shown in FIG. 70, a structure is provided in which the cutting electrode 6 is disposed so as to traverse the first lumen 3, and as shown by the broken line in FIG. 70, the cutting electrode 6 (the activation part of the device) projects from the distal end opening 3A so as to be usable. In this state, as shown in FIGS 71 and 72, the fastening screw 129a of the fastening member 129 of the electrode controlling portion 128 is loosened, and among the electrode tubes 18a and 18b, which are a pair of electrode lumens, the proximal side end portion of the one electrode tube 18b is detached from the fastening member 129. Thereby, the electrode controlling wire 7B is exposed between the proximal end opening of the electrode tube 18b and the fastening member 129.

While the electrode controlling wires 7A and 7B (operation part of the device) are fastened to the electrode controlling portion 8, as shown in FIGS. 72 and 73, the electrode control portion 128 and the electrode tube 18b are moved in a direction of relative separation such that the electrode control wire 7B inside the electrode tube 18b is moved to retract back in the proximal direction of the overtube 130 with respect to the electrode tube 18b.

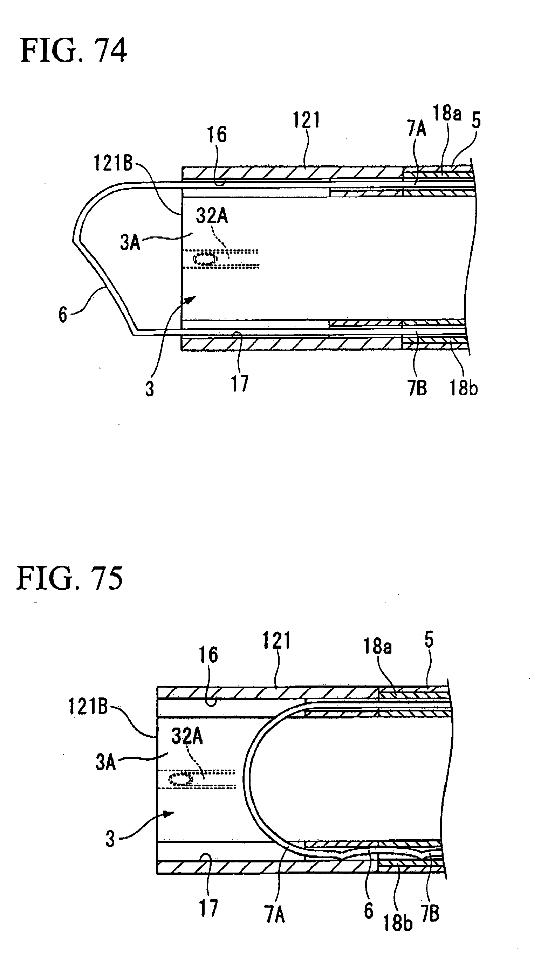

As shown in FIG 74, the electrode controlling wire 7B is drawn inside through the distal end opening of the electrode tube 18b, and the portion coupled to the electrode control wire 7B is drawn back toward the proximal end side by one end portion of the cutting electrode 6. While maintaining the state of separation between the electrode controlling portion 128 and the electrode tube 18b, when the entire electrode controlling portion 128 has been retracted, the cutting electrode 6 is drawn into the electrode tube 18b by an amount equivalent to the amount that the electrode controlling wire 7B has been drawn, and as shown in FIG 75, the cutting electrode 6 is accommodated in the electrode tube 18b.

When the cutting electrode 6 is exposed, the electrode controlling wires 7A and 7B are fastened, and the electrode controlling portion 128 and the electrode tube 18b are moved relatively so as to approach each other such that the electrode controlling wire 7B in the electrode tube 18b is pressed in the distal direction of the overtube 130 with respect to the electrode tube 18b. Thereby, the cutting electrode 6 is exposed from inside the electrode tube 18b, and subsequently, the electrode tube 18b is again fastened to the electrode controlling portion 128.

By carrying out the control in this manner, the advancement and retraction control (and protection) of the cutting electrode 6 is possible. Note that the electrode tube 18b is also structured so as to be freely releasably attached in the fastening member 29, and by pulling either of the electrode controlling wires 7A and 7B, the cutting electrode 6 may be accommodated in the corresponding electrode tube 18a or 18b.

In addition, in other methods as well, for example, in the case of accommodating the cutting electrode 6, the operation of pressing the control handle 21, and the operation of moving the electrode tube 18b may be carried out simultaneously.

Furthermore, as another method, for example, the control handle 21 may be structured by a first control handle and a second control handle that can be operated relative to each other, and the first control handle may be connected to the electrode controlling wire 7A and the second control handle may be connected to the electrode control wire 7B. In this case, when the two control handles are operated in mutually opposing directions, it is possible to carry out the advancement and retraction control of the cutting electrode 6 (i.e., the operation in which the cutting electrode 6 is accommodated in the electrode tubes 18a and 18b and the operation in which the cutting electrode 6 is exposed from the electrode tubes 18a and 18b). In addition, in order to cut the tissue, while the cutting electrode 6 is disposed so as to traverse the first lumen 3, when the cutting electrode 6 is advanced and retracted relative to the overtube 1, the first controlling handle and the second controlling handle are operated in the same direction simultaneously.

The scope of the art of this invention is not restricted to the embodiments described above, and various changes can be added within a range that does not fall outside the spirit of this invention.

For example, though in each of the above-described embodiments, a flexible endoscope is used as the observation device, this invention is not restricted thereto and, for example, a so-called capsule endoscope may be placed inside the body, and while observing the interior of the body using the endoscope, an insertion part of a treatment device that does not have an observation device may be inserted through the overtube to perform the desired surgical procedure.

Though in the first embodiment, the incision electrode 6 is set to a length such that it is accommodated inside the lumen 3 in a bended state as shown in FIG. 4, this invention is not restricted thereto, and the incision electrode 6 may be set to a length such that it is accommodated inside the lumen 3 without being bended. The length of the incision electrode (length of the portion that crosses the lumen) may be set suitably according to the outer diameter of the overtube itself or according to the outer diameter of a device that is inserted through the lumen. This prevents the forming of an opening that is greater than necessary. Thus when a device is introduced into the abdominal cavity through an opening in a hollow organ that has been formed using the incision electrode, the gap formed between the device and the opening can be held at the minimum and sealing of the inner side and the abdominal cavity side of the hollow organ can be secured at a level by which the pressure applied to the abdominal cavity AC can be maintained.

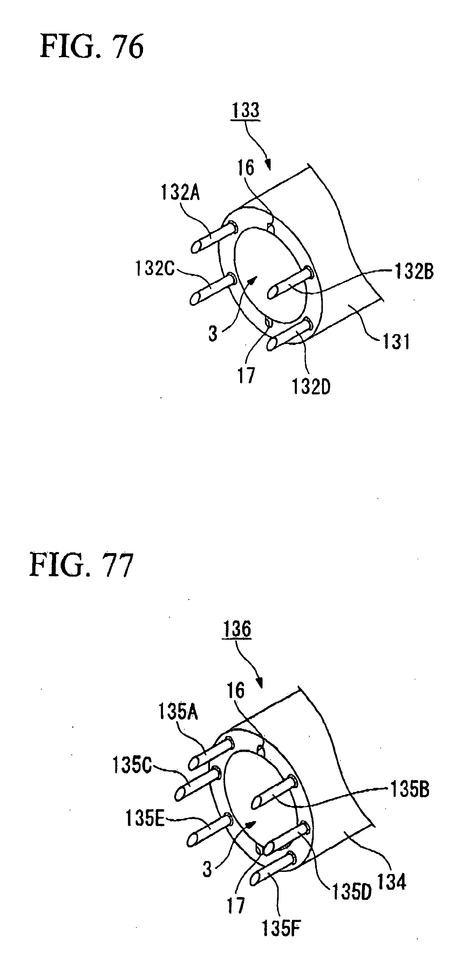

An overtube 133, having four puncture needles 132A, 132B, 132C, and 132D disposed at a distal end part 131, may be arranged as shown in FIG 76. In this case, the puncture needles 132A and 132B and the puncture needles 132C and 132D are positioned at respectively symmetrical positions with respect to a line joining the first inner groove 16 and the second inner groove 17 of the distal end part 131. By housing the anchors 33A of the double T-bars 33 in the respective puncture needles, two suturing locations can be secured with respect to the incision direction.

Likewise, an overtube 136, having six puncture needles 135A, 135B, 135C, 135D, 135E, and 85F disposed at a distal end part 134, may be arranged as shown in FIG. 77. In this case, the puncture needles 135A and 135B, the puncture needles 135C and 135D, and the puncture needles 135E and 135F are positioned at respectively symmetrical positions with respect to a line joining the first inner groove 16 and the second inner groove 17 of the distal end part 134. By housing the anchors 33A of the double T-bars 33 in the respective puncture needles, three suturing locations can be secured with respect to the incision direction.

As shown in FIGS. 76 and 77, by providing four or six puncturing needles at the distal end part and thereby securing a plurality of suturing locations with respect to the incision location, a more reliable suturing can be carried out.

As shown in FIGS. 76 and 77, a scale 141 may be provided on the side portion of the proximal handle 140 along the movement path of the bending control lever 45. By confirming the scale 141, the condition of the bending can be easily grasped. Furthermore, a scale detecting apparatus 142 may be provided The scale detecting apparatus 142 has a first lever 143 and a second lever 144 disposed so as to surround the operation direction of the bending control lever 45. The first lever 143 provides an engaging piece 143A that can abut the side surface 45A of the curvature controlling levers 45. The second lever 144 provides an engaging piece 144A that can abut the other side surface 45B of the bending control lever 45. The respective levers 143 and 144 are provided freely rotatably and coaxial to the bending control lever 45. An example of a supporting structures for the levers 143 and 144 are a rotating axle of the bending control lever 45, a tube separately inserted freely rotatably between the levers 143 and 144 and the cover of the proximal handle 140, and a flexible member that is fastened in the tube and that produces a prescribed frictional force between the levers 143 and 144 and the cover of the proximal handle 140.

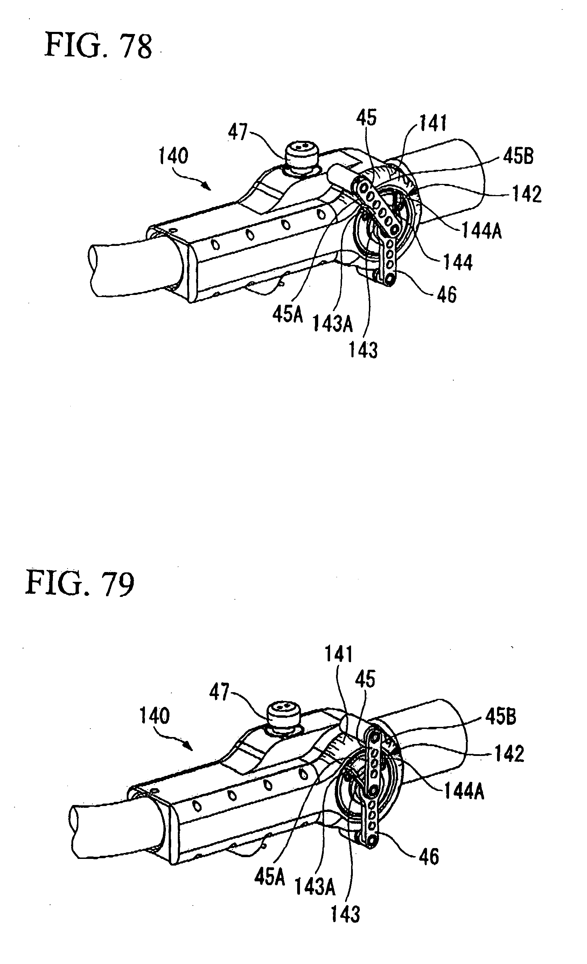

In FIG. 78, the curvature control lever 45 is inclined at a first angle. From this state, as shown in FIG. 79, when the curvature control lever 45 is rotated, the engagement of the first lever 143 with the engaging piece 143A is released. Because the first lever 143 is provided separately from the curvature control lever 45 and is supported by a prescribed frictional force on the proximal handle 140, the first lever 143 stops and remains at the first angle. Even when the curvature control lever 45 is returned, it is possible to confirm the first angle from the position of the first lever 143.

An example of the usage of such a scale detection apparatus 142 is switching the endoscope 2 after introducing the overtube 1 and the endoscope 2 into the body and bending them to a first angle. When the bent portion 13 of the overtube 1 is greatly bent (that is, when bent to a small radius of curvature), it is then possible to replace the endoscope 2, and the replacement action can be carried out smoothly after the bent portion 13 has temporarily returned to a gently bent shape or a rectilinear shape.

After inserting a new endoscope 2, if the curvature control lever 45 is returned to the position of the first lever 143, the curvature angle that was determined first, that is the position of the distal end portion, reappears. Note that in the meantime, the second lever 144 does not engage with the curvature control lever 45, and stops in the initial position. When the overtube 1 is bent toward the opposite side, the second lever 144 is operated as described above.

In addition, by operating the curvature control lever 45, the bent portion 13 is provided in two directions (two directions, wherein the insertion portion 5 is opposite to the substantially rectilinear state). However, two curvature control levers 45 may be provided to enable bending in 4 directions.

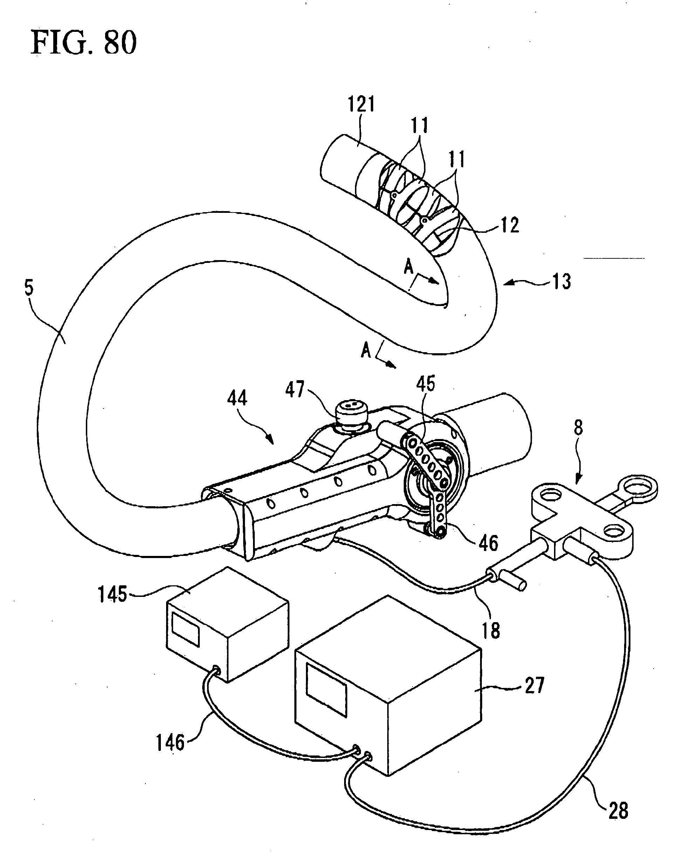

As shown in FIG 80, an impedance measuring device 145 may also be connected by a cable 146 to a high frequency power source 27, which is connected to the terminal of the electrode controlling portion 8 via the power cord 28. Thereby it is possible to detect that the cutting of the tissue has been completed.

The impedance measuring device 145 is a device that detects fluctuations in the impedance of the cutting electrode 6 when the high frequency power source 27 energizes the cutting electrode 6. It is known that generally, when the tissue is cauterized, the impedance becomes high. Thus, the impedance before cutting the tissue is found in advance. A rise in the impedance to a maximum is considered to signify that the cutting of the tissue has been completed, and the energizing of the cutting electrode 6 is stopped. Alternatively, when the cutting has been completed, the impedance falls, Thus, the timing at which the impedance begins to fall can be considered to signify that the cutting of the tissue has completed, and the energizing of the cutting electrode 6 may be stopped. Thereby, excess energizing can be prevented, and it is possible to cut only the required part.

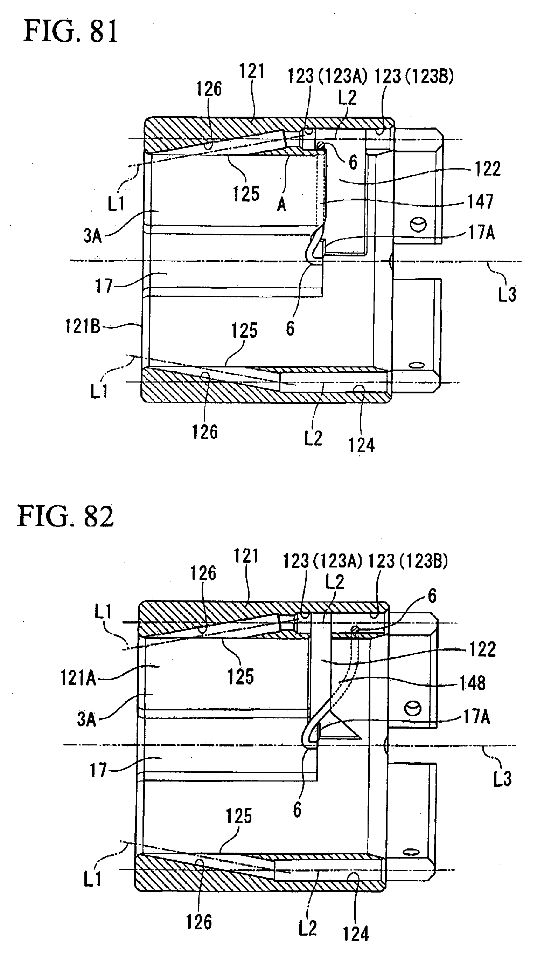

As shown in FIG 81, a hook-shaped holding portion 147 (expanded portion) may be formed by extending a portion of the inner peripheral portion of the distal end side that forms the accommodation portion 122 so as to cover the accommodation portion 122 substantially parallel to the axial line L3. By hooking the cutting electrode 6 in the holding portion 147, the cutting electrode 6 can be accommodated in the accommodation portion 122 irrespective of the state of the puncture needle 32A. When the cutting electrode 6 is used, the engagement is released by pushing and pulling the electrode controlling wires 7A and 7B.

As shown in FIG 82, a hook-shaped holding portion 148 (expanded portion) may be formed by extending a portion of the inner peripheral portion of the proximal end side that forms the accommodation portion 122 so as to cover the accommodation portion 122 substantially parallel to the axial line L3. By hooking the cutting electrode 6 in the holding portion 148, the cutting electrode 6 can be accommodated in the accommodation portion 122 irrespective of the state of the puncture needle 32A. When the cutting electrode 6 is used, the engagement is released by pressing the electrode controlling wires 7A and 7B.

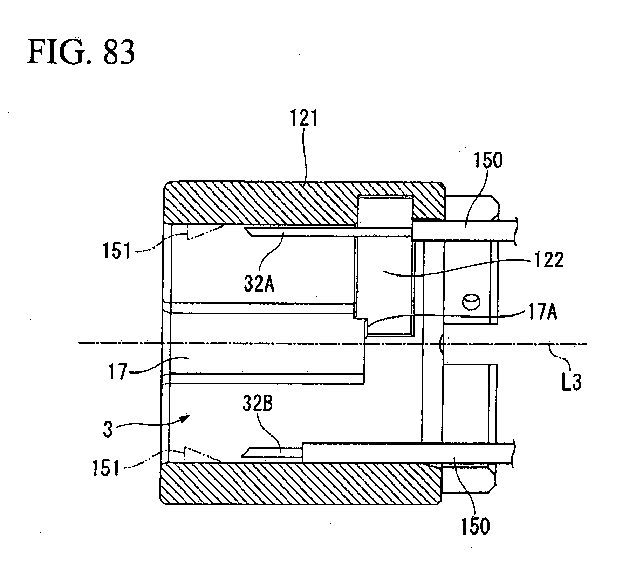

As shown in FIG 83, a needle lumen 150 (second lumen) may be fastened along the inner surface that forms the first lumen 3. Puncture needles 32A and 32B may each pass so as to be freely extendable and retractable in the needle lumen 150. Because it is possible to enter tissue that is drawn from the distal end opening of the first lumen 3, effects identical to those described above can be obtained. In this case, as shown by the imaginary line in FIG. 83, when a projection shaped or taper shaped guide portion 151 that projects on the distal end or inside is provided on the inner periphery of the distal end portion 121 on the movement path of the puncture needles 32A and 32B, because the puncture needles 32A and 32B are urged inside by the guide portion, it is possible to set the entrance position more toward the center.

## Claims

1. An overtube **characterized by** comprising:
an insertion part, that is inserted into a subject and has a lumen, through which a device insertion part of a device for performing a medical procedure inside a body of the subject is removably inserted, the insertion part being inserted into the subject; and
a tissue incising part that is disposed at a distal end side of the insertion part so as to cross the lumen and incises a tissue of the subject.

2. The overtube according to claim 1, wherein said tissue incising part is disposed so as to allow withdrawing of the crossing state of said lumen.

3. The overtube according to claim 2, wherein said tissue incising part can be removed from the insertion part.

4. The overtube according to claim 1, further comprising:
a manipulating member, connected to the tissue incising part and being disposed in a manner enabling advancing and retracting with respect to the insertion part; and
a manipulating part for manipulating the manipulating member to advance and retract with respect to the lumen.

5. The overtube according to claim 4, wherein the manipulating member is detachably connected to the manipulating part.

6. The overtube according to claim 1, wherein the tissue incising part crosses a center of the lumen from an outer edge of the lumen in a direction orthogonal to an axial direction of the insertion part.

7. The overtube according to claim 1, wherein hollow puncture needles, advancing and retracting along the lumen, are disposed at the distal end side of the insertion part.

8. The overtube according to claim 7, further comprising:
thread members extending from the tissue incising part to a proximal end of the insertion part, and
a fastening member provided on distal ends of the thread members for fastening the thread members to a tissue of the subject in proximity to a targeted incision site.

9. The overtube according to claim 8, wherein said fastening member comprises anchors attached to the distal ends of the thread members.

10. The overtube according to claim 9, wherein said anchors are respectively held inside the puncture needles.

11. The overtube according to claim 10, wherein two sets of the thread member in which the anchors attached to the distal end sides thereof, and two sets of said puncture needle are provided; and each of the two anchors retains on both sides of the targeted incision cite so as to situate the targeted incision cite therebetween.

12. The overtube according to claim 11, further comprising a member for adjusting a gap between the anchors which are attached to the distal ends of the thread members by decreasing the gap to the predetermined value.

13. The overtube according to claim 12, wherein said cutting instrument uses a high frequency.

14. The overtube according to claim 12, wherein the cutting instrument has a tip, and a a radius of curvature of the tip is set within a range of 0.05 mm to 0.5 mm.

15. The overtube according to claim 7, wherein said puncture needle can be protrude out from an inside of the lumen and can be observed by an observation apparatus which is provided at the distal end side of the lumen.

16. The overtube according to claim 1, wherein a magnetic body is disposed in the insertion part.

17. The overtube according to claim 1, further comprising a holding portion that holds the tissue cutting portion at a position that is removed from a position traversing the first lumen.

18. The overtube according to claim 17, wherein the holding portion includes puncture needles that are disposed so as to be freely advanced and retracted in the insertion portion and that are to puncture the tissue.

19. The overtube according to claim 18, wherein the distal end portion of the insertion portion has a groove provided therein, and the tissue cutting portion is engaged so as to be retained in the groove by the puncture needle that traverses the groove.

20. The overtube according to claim 19, wherein the puncture needles are structured to accommodate anchors that are to be retained in the tissue.

21. The overtube according to claim 17, further comprising an expanded portion, wherein the expanded portion has a groove that is provided in the distal end portion of the insertion portion, and the holding portion is formed by extending an inner peripheral side of the distal end portion of the insertion portion so as to cover a portion of the groove of the expanded portion.

22. the overtube according to claim 17, wherein the holding portion includes a tube that extends from one end side and the other end side of the tissue cutting portion and that passes through an operating member that extends toward the proximal end portion of the insertion portion by passing through the insertion portion.

23. The overtube according to claim 17, wherein said manipulating member moves the tissue cutting portion from a first position removed from the position traversing the lumen to a second position traversing the lumen.

24. The overtube according to claim 23, wherein said puncture needle is provided in an inside of said overtube that is defined by said lumen, and the overtube further comprising a needle manipulating part for retracting said puncture needle to the proximal end direction of said insertion portion.

25. The overtube according to claim 23, wherein a tube that passes through the insertion portion is provided, and the tissue cutting portion which is held in the tube can be advanced with respect to the tube.

26. The overtube according to claim 1, further comprising:
a first lumen that is formed in the insertion portion and that allows freely inserting and removing a device for carrying out medical procedures in the subject;
puncture needles that pass through the insertion portion, have a control member connected to the proximal end portion of the insertion portion, and puncture tissue; and
second lumens that are provided in the insertion portion and through which puncture needles are passed;
wherein the second lumens having on the distal end portion of the insertion portion openings through which distal ends of the puncture needles extend and retract, and the openings being provided on an inside of the insertion portion defined by the first lumen.

27. The overtube according to claim 26, wherein an opening is formed on the distal end portion of the insertion portion so as to communicate with the first lumen in order to project the device in the forward insertion direction for insertion into the subject.

28. The overtube according to claim 27, further comprising a guide portion that controls the projection direction of the puncture needles that project from the openings of the second lumens and that makes the puncture needles project in a center direction of the first lumen that is in cross-section substantially perpendicular to the direction in which the distal end portion of the insertion portion connects the proximal end portion of the insertion portion.

29. The overtube according to claim 27, wherein, at the distal end portion of the insertion portion, the second lumens are formed inside the distal end portion, and the centers of the openings of the second lumens are formed in the inner surface of the distal end portion of the first lumen more towards the proximal side than the opening of the insertion portion.

30. The overtube according to claim 29, wherein a distal end area of the second lumens has a passage that angles the puncture needles from the proximal end portion to the distal end portion from the inner surface of the distal end portion that forms the first lumen to the direction of the center axis of the first lumen.

31. The overtube according to claim 26, wherein an abutting portion formed at a distal end of the distal end portion of the insertion portion abuts the tissue when the tissue of the subject is sucked.

32. The overtube according to claim 31, wherein each puncture needle has a passage therein that can accommodate an anchor that is to be disposed on an exit side of a punctured tissue.

33. The overtube according to claim 32, wherein the puncture needles include a first puncture needle and a second puncture needle, each of the puncture needles being disposed at opposing positions across the approximate center of the first lumen.

34. The overtube according to claim 33, wherein the anchors accommodated in the first puncture needle and the second puncture needle are connected together by a thread, and the distal end has a hole which accommodates a stopper portion provided on the thread.

35. The overtube according to claim 1, further comprising: a first lumen that is formed in the insertion portion that has a distal end portion and a proximal end portion, and allows freely inserting and removing a device for carrying out medical procedures in the subject;
puncture needles that pass through the insertion portion, have a control member connected to the proximal end portion of the insertion portion, and puncture tissue; and
second lumens that are provided in the insertion portion and through which puncture needles are passed, the second lumens having on the distal end portion of the insertion portion openings through which, distal ends of the puncture needles extend and retract, and the openings being provided on an inside of the insertion portion which forms the first lumen.

36. The overtube according to claim 1, further comprising:
an insertion portion having a distal end portion and a proximal end portion and at least the distal end portion thereof is inserted into an examination subject;
a pair of lumen tubes formed in the insertion portion, each of the pair of lumen tubes having a distal end opening and a proximal end opening formed therein, and disposed such that the distal end opening is separated at the distal end portion of the insertion portion;
an activation part disposed between the respective distal end openings of the pair of lumen tubes so as to be usable;
a device that extends from both ends of the activation part and has an operation part that passes through the pair of lumen tubes so as to freely advance and retract; and
a fastening member that releasably fastens proximal end portions of the pair of lumen tubes that are pulled from the proximal end portion of the insertion portion, and while the operation part is exposed by releasing one of the pair of lumen tubes, the released lumen tube can be moved relative to the operation part.
